# EUROPEAN PATENT SPECIFICATION

(11) **EP 3 651 860 B1**
(45) Date of publication and mention of the grant of the patent: **04.03.2026**
(21) Application number: 18749578.3
(22) Date of filing: 11.07.2018
(51) Int. Cl.: A61Q 19/04, A61K 8/97, A61K 8/63, A61Q 19/02, A61K 8/9783, A61Q 19/00, A61K 8/9789

(54) **AGENT FOR PREVENTING OR AMELIORATING UNEVEN SKIN TONE**
MITTEL ZUR VERMEIDUNG ODER MINDERUNG EINES UNEBENMÄSSIGEN HAUTTONS
AGENT DE PRÉVENTION OU D'AMÉLIORATION DE TEINT DE PEAU IRRÉGULIER

(30) Priority: 12.07.2017 US 201762531570 P; 09.07.2018 US 201816030405
(43) Date of publication of application: 20.05.2020
(73) Proprietor: Kao Corporation, Chuo-Ku Tokyo 103-8210 (JP)
(72) Inventor: MURASE, Daiki, Haga-gun Tochigi 321-3497 (JP); KUSAKA, Ayumi, Odawara-shi Kanagawa 250-0002 (JP); HACHIYA, Akira, Haga-gun Tochigi 321-3497 (JP); IMAI, Asuka, Cincinnati, Ohio 45214 (US)
(74) Representative: Hoffmann Eitle
(86) International application number: PCT/JP2018/026245
(87) International publication number: WO 2019/013270

(56) References cited:
- CN-A- 105 012 820
- US-A1- 2011 117 038
- US-B2- 8 927 517

## Description

### [Technical Field]

The present invention relates to an in vitro method for evaluating or selecting an agent for preventing or ameliorating uneven skin tone and to a non-therapeutic method for preventing or ameliorating uneven skin tone.

### [Background Art]

Joint sites such as elbow, knee, and finger joint have excess portions in their skin (portions which slightly sag when stretching the joints) to enable stretching and bending motions and are often subjected to friction and load during ordinary living. Therefore, damage such as discoloration and drying tends to occur in the skin over the joint sites, and uneven skin tone thereby tends to occur between the joints and their peripheries.

The state of the uneven skin tone observed at the joints is particularly remarkable at the joint sites of races with dark skin tone such as African races. Further, the uneven skin tone of the races with dark skin tone has a characteristic appearance called ashy skin. Ashy skin is a skin which appears to be powder-dusted (ashy) due to dryness. Therefore, the uneven skin tone seen at the joint sites of the races with dark skin tone is often accompanied by a decrease in skin saturation due to ashy skin in addition to skin darkening (decrease in skin brightness due to pigmentation). The uneven skin tone accompanied by ashy skin is a big cosmetic problem for the races with dark skin tone, particularly, African races. However, the details of uneven skin tone and ashy skin have not been revealed so far, and an effective approach for ameliorating uneven skin tone and ashy skin has not been proposed yet.

Melanin is a pigment present in human skin. Melanin is biosynthesized in melanocytes in the basal layer of epidermis and is sent to keratinocytes via dendrites extending from melanocytes. When melanin accumulates in keratinocytes, the skin darkens. In recent years, it has been found that autophagy is involved in metabolism of melanin in keratinocytes. The skin tone can be brightened by activating the autophagy pathway in keratinocytes and thereby reducing the amount of melanin in keratinocytes (Patent Literature 1). However, in order to deal with uneven skin tone accompanied by a decrease in saturation in addition to skin darkening, such as uneven tone in ashy skin, the approach by melanin control alone is not enough.

PTL 2 refers to dopa oxidase inhibitor, a skin-lightening agent and an external preparation for skin, comprising an extract of at least one plant selected from the group consisting of Melia toosendan Sieb. et Zucc., Amomum tsao-ka Crevost et Lemaire, Senecio gracilis and Veratrum nigrum L. as an active ingredient.

PTL 3 relates to a special Chinese herb preparation for treating parasitic amassment, expelling parasites and treating face vitiligo and a preparation method thereof.

### [Citation List]

### [Patent Literature]

[PTL 1] U.S. Patent No. 8,927,517
[PTL 2] US 2011/117038 A1
[PTL 3] CN 105 012 820 A

### [Summary of the Invention]

The present invention provides an in vitro method for evaluating or selecting an agent for preventing or ameliorating uneven skin tone, the method comprising: administering test substances to cells; measuring changes in autophagic activity caused by the test substances in the cells; and selecting a test substance which has enhanced the autophagic activity.

Further, the present invention provides a non-therapeutic method for preventing or ameliorating uneven skin tone, the method comprising: enhancing autophagic activity in keratinocytes in skin in need thereof, wherein the uneven skin tone is skin discoloration with skin dryness, thickening, and pigmentation, or ashy skin with pigmentation, wherein the uneven skin tone is uneven tone in skin over joints, and wherein the enhancement of the autophagic activity in keratinocytes in the skin comprises administering at least one selected from the group consisting of Melia toosendan, its extract, and toosendanin to the skin.

Further, the present invention provides a non-therapeutic method for preventing or ameliorating uneven skin tone, the method comprising: administering at least one selected from the group consisting of Melia toosendan, its extract, and toosendanin to skin in need thereof, wherein the uneven skin tone is skin discoloration with skin dryness, thickening, and pigmentation, or ashy skin with pigmentation, wherein the uneven skin tone is uneven tone in skin over joints.

Further, the present invention provides a non-therapeutic use of senrenshi (Melia Toosendan), its extract or toosendanin for preventing or ameliorating uneven skin tone, wherein the uneven skin tone is skin discoloration with skin dryness, thickening, and pigmentation, or ashy skin with pigmentation, wherein the uneven skin tone is uneven tone in skin over joints.

### [Brief Description of the Drawings]

[Fig.1] Uneven skin tone (ashy skin) of African American (AA) .
   a: Images of AA and Caucasian elbows.
   b: Images of skins of elbows and upper outer and inner arms of AA and Caucasian.
   PL: Polarized images.
   NPL: Non-polarized images.
[Fig.2] Skin tone parameters measured for the upper outer arm and elbow skins of AA.
   Error bar = SD (n = 12), *: p < 0.05, **: p < 0.01, ***:
   p < 0.0001 (paired t-test).
[Fig.3] a: Stratum corneum water content (Capacitance) and trans-epidermal water loss (TEWL) of the upper outer arm and elbow skins of AA.
   Error bar = SD (n = 12), *: p < 0.05, **: p < 0.01, ***:
   p < 0.0001 (paired t-test).
   b: Correlations of stratum corneum water content
   (Capacitance) and trans-epidermal water loss (TEWL) with skin saturation (C*).
   p < 0.05 (Regression analysis)
[Fig.4] Images of skin surfaces, melanin-stained images of skin sections, and immunohistochemically stained images of skin sections of Caucasian, from the top, with white dot lines in the immunohistochemically stained images indicating the boundary between the epidermis and the dermis or the outermost layer of the stratum corneum.
[Fig.5] Images of skin surfaces, melanin-stained images of skin sections, and immunohistochemically stained images of skin sections of AA, from the top, with white dot lines in the immunohistochemically stained images indicating the boundary between the epidermis and the dermis or the outermost layer of the stratum corneum.
[Fig.6] Quantitative analysis results of the immunohistochemically stained images in Figures 4 and 5. IA: Upper inner arm, OA: Upper outer arm, E: Elbow, **: p < 0.01, *: p < 0.05 (ANOVA, Bonferroni test).
[Fig.7] Immunohistochemically stained images of Caucasian skin sections, with white dot lines in the immunohistochemically stained images indicating the boundary between the epidermis and the dermis or the outermost layer of the stratum corneum.
[Fig.8] Immunohistochemically stained images of AA skin sections with white dot lines in the immunohistochemically stained images indicating the boundary between the epidermis and the dermis or the outermost layer of the stratum corneum.
[Fig.9] Quantitative analysis results of the immunohistochemically stained images in Figures 6 and 7. IA: Upper inner arm, OA: Upper outer arm, E: Elbow, **: p < 0.01, *: p < 0.05 (ANOVA, Bonferroni test).
[Fig.10] Autophagic activities in AA skins.
   a: Western blotting results for LC3-II.
   b: Quantitative values of autophagic activity.
   Error bar = SD (n = 5), **: p < 0.01 (paired t-test).
   c: Correlations of autophagic activity with skin saturation (C*), stratum corneum water content (Capacitance), and trans-epidermal water loss (TEWL).
   *: p < 0.05 (Regression analysis).

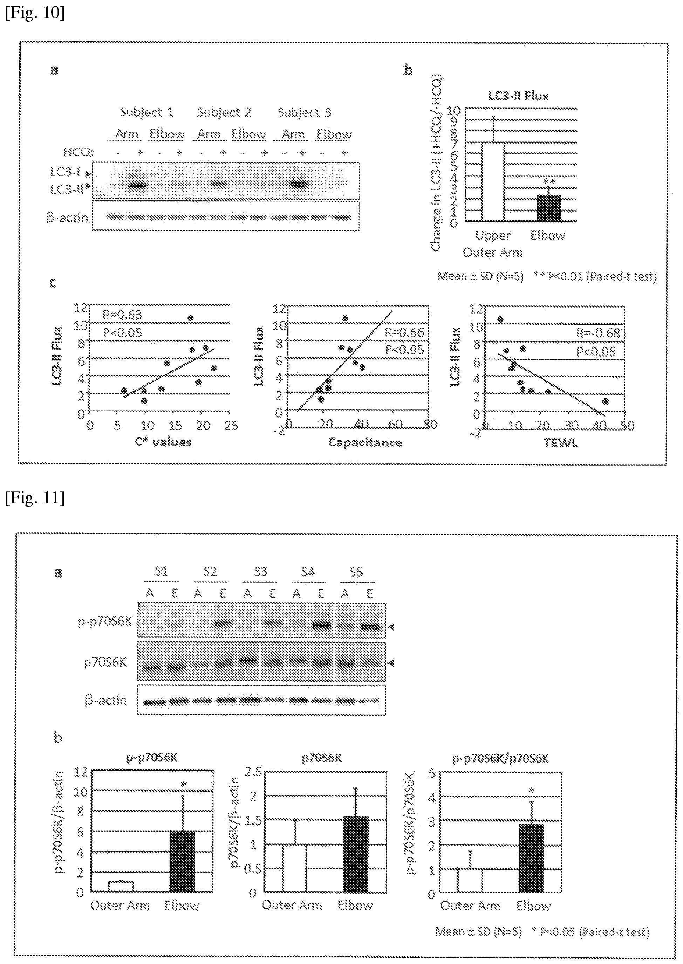

[Fig.11] mTORC1 activities in AA skins.
   a: Western blotting results for phosphorylated p70S6K (p-p70S6K) and non-phosphorylated p70S6K.
   A: Upper outer arm, E: Elbow.
   b: Quantitative values of p-p70S6K and p70S6K, and their ratio.
   Error bar = SD (n = 5), *: p < 0.05 (paired t-test).

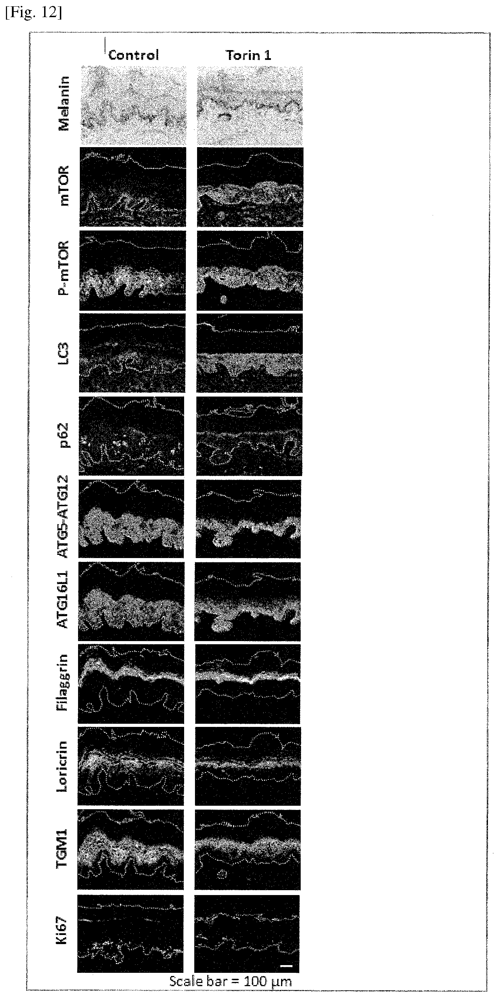

[Fig.12] Enhancement in autophagic activities in Torin 1-treated AA skin.
   Melanin-stained images of skin sections and immunohistochemically stained images of autophagy-related proteins, from the top, with white dot lines in the immunohistochemically stained images indicating the boundary between the epidermis and the dermis or the outermost layer of the stratum corneum.
   Control: Skin without Torin 1 treatment, Torin 1: Torin 1-treated skin.

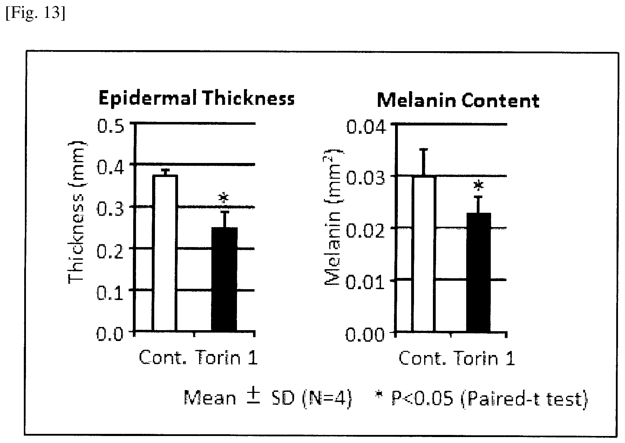

[Fig.13] Reductions in epidermal stratum corneum thickness and melanin amount of Torin 1-treated AA skin Cont.: Skin without Torin 1 treatment, Torin 1: Torin 1-treated skin, Error bar = SD (n = 4), *: p < 0.05 (paired t-test).

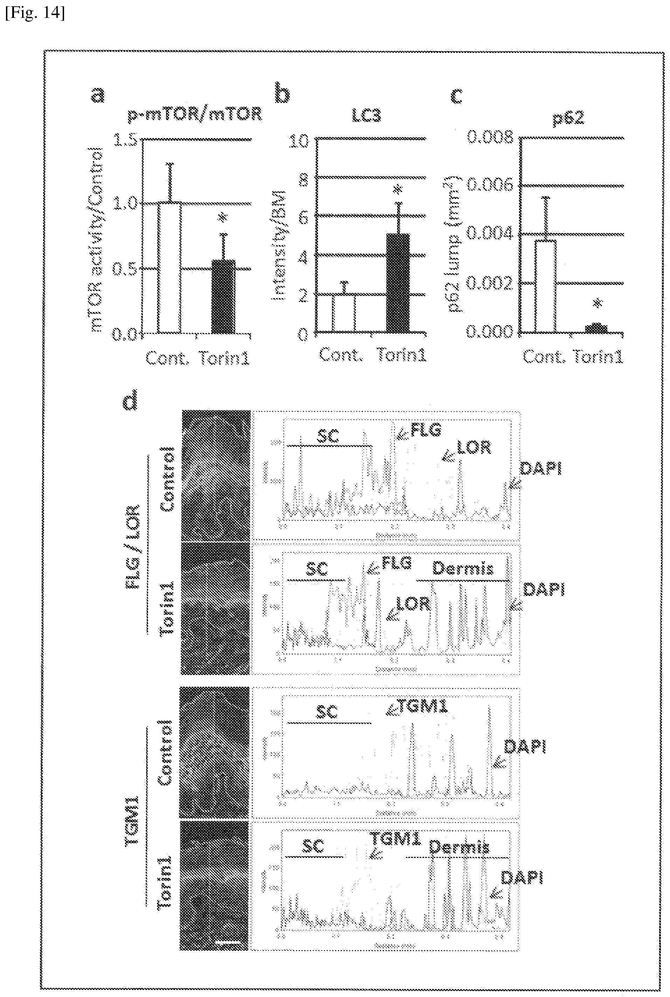

[Fig.14] Enhancement in autophagic activity in Torin 1-treated AA skin.
   a: Ratios of quantitative values of phosphorylated mTOR. (p-mTOR) to mTOR expression.
   b: Ratios of quantitative values of LC3 expression.
   c: Quantitative values of p62 expression.
   Control: Skin without Torin 1 treatment Torin 1: Torin 1-treated skin, Error bar = SD (n = 4), *: p < 0.05 (paired t-test).
   d: RGB Profile Plot.

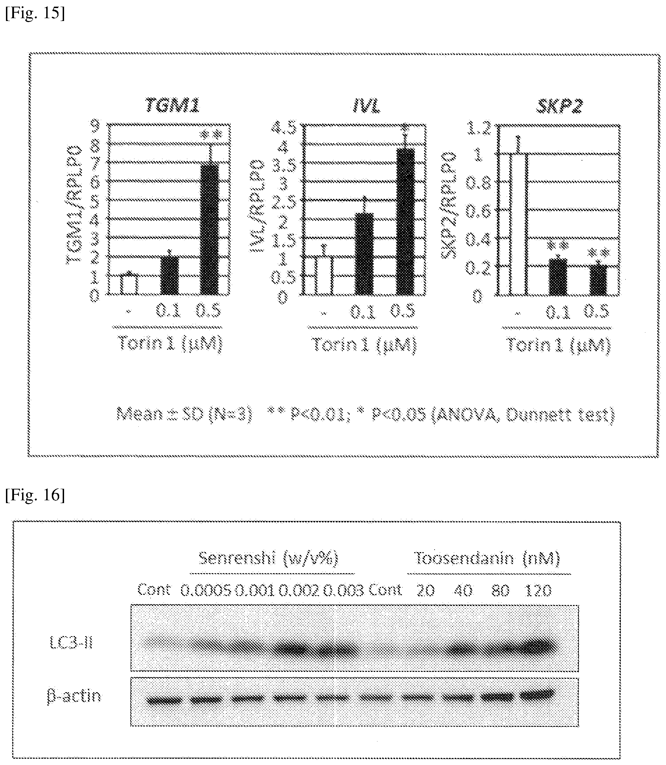

[Fig.15] Enhancement in gene expressions of keratinization-related molecules and cell proliferation-related molecules in Torin 1-treated AA keratinocytes. Error bar = SD (n = 3), *: p < 0.05 (ANOVA, Dunnett test)
[Fig.16] Enhancement in autophagic activity in keratinocytes by senrenshi extract and toosendanin as Western blotting results for LC3-II, with
   the concentration of senrenshi extract being a solid content concentration.

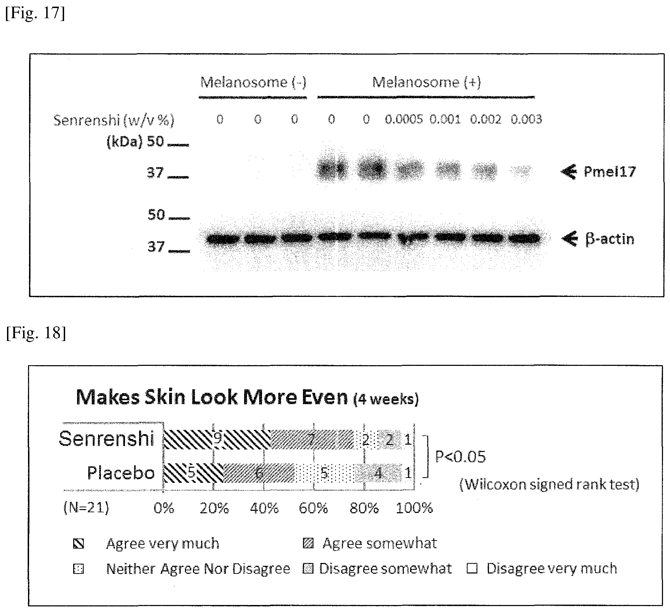

[Fig.17] Melanosome degradation activity of senrenshi extract as Western blotting results for Pmel17, with the concentration of senrenshi extract being a solid content concentration.
[Fig.18] Ameliorating Action of senrenshi extract on uneven skin tone as questionnaire results from test subjects.

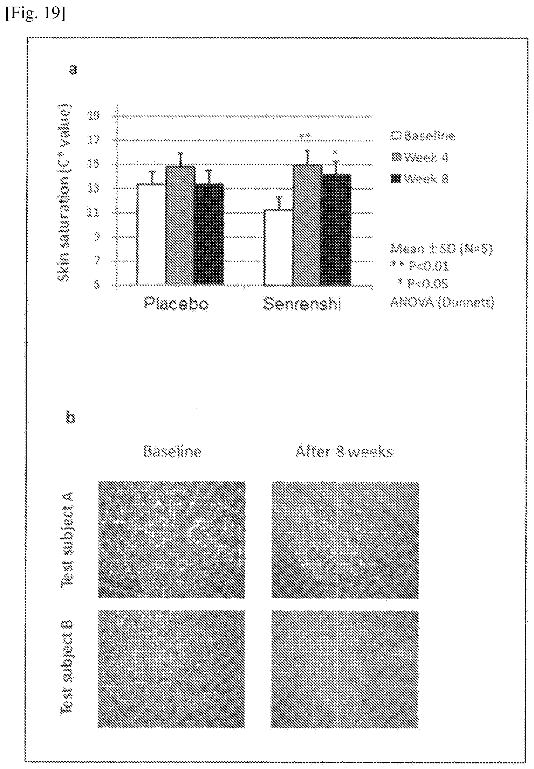

[Fig.19] Ameliorating Action of senrenshi extract on uneven skin tone.
   a: Skin saturation (C*).
   Error bar = SD (n = 5), *: p < 0.05, **: p < 0.01 (ANOVA, Dunnett test).
   b: Images of skin surfaces.
   Error bar = SD (n = 5), *: p < 0.05, **: p < 0.01 (ANOVA, Dunnett test).

### [Detailed Description of the Invention]

### (1. Definition)

In this specification, the "identity" relating to amino acid sequences or nucleotide sequences means a ratio (%) of the number of positions where identical amino acid residues or nucleotides are present in both of two amino acid sequences or nucleotide sequences when the two sequences are aligned (in alignment), with respect to the number of full-length amino acid residues or nucleotides. Specifically, it is a value calculated by the Lipman-Pearson method (Science, 227, 1435, 1985) by analysis using a homology analysis (Search homology) program of a genetic information processing software, Genetyx-Win (Ver.5.1.1; software development), with a unit size to compare (ktup) being set to 2.

Autophagy is a mechanism of cellular molecules to degrade cellular components by lysosomes. Autophagy was first found as a mechanism to degrade unnecessary proteins for cells, but actually the objects to be degraded by autophagy are not limited to proteins, and large structures such as organelles are also degraded. Autophagy is composed of a series of processes occurring through special organelles called autophagosomes. In the autophagy pathway, an isolating membrane or a small vesicle called phagophore is first generated in cytoplasm. The phagophore gradually extends to engulf around part of the cytoplasm, and then forms an autophagosome which is a double-membrane bag-like structure. Subsequently, the outer membrane of the autophagosome fuses with a lysosome to form an autolysosome, in the course of which substances thereinside are degraded together with the inner membrane of the autophagosome. The degradation products generated by autophagy are reused in cells. In the autophagy pathway, a plurality of steps such as phagophore generation and extension, autophagosome formation, fusion between autophagosomes and lysosomes, and autolysosome formation, and degradation of internal substances intervene, and a large number of factors are involved in each step. The main factors are proteins collectively referred to as ATG (autophagy-related proteins) and involved in phagophore and autophagosome formation. 30 types or more of ATG have been reported so far.

In this specification, the "autophagy-related genes" can be genes encoding proteins involved in each step of the autophagy pathway, such as phagophore generation, phagophore extension or growth, autophagosome formation, fusion between autophagosomes and lysosomes, autolysosome formation, and degradation of substances inside autolysosomes. Further, in this specification, examples of the "autophagy-related molecules" include molecules (such as proteins and mRNA) encoded by the autophagy-related genes involved in each step of the autophagy pathway. Alternatively, examples of the autophagy-related molecules include molecules involved in phagophore generation, molecules involved in phagophore growth or autophagosome formation, and molecules involved in fusion between autophagosomes and lysosomes, which will be described below. In this specification, the autophagy-related genes or the autophagy-related molecules may be hereinafter referred to collectively as "autophagy-related genes or molecules".

Examples of the molecules involved in phagophore generation in mammalian autophagy include ATG1 (or ULK1 or ULK2), ATG13, ATG17, ATG29, ATG31, ATG101, FIP200, VPS34, VPS15 (or p150), ATG6 (or VPS30 or Beclin1), ATG14, Ambra1, ATG2, ATG9, ATG18 (or WIPI1-4), DFCP1, and VMP1. Among these, ATG1 (or ULK1 or ULK2), ATG13, ATG17, ATG29, ATG31, ATG101, and FIP200 are considered to be involved in initial phagophore formation as ULK/Atg1 complex. Meanwhile, VPS34, VPS15, ATG6, ATG14, and Ambra1 produce PI(3)P (phosphatidylinositol 3-phosphate) mainly in the endoplasmic reticulum as Class III PI3-kinase complex. Further, ATG2 and ATG18 have a PI(3)-P binding ability and are involved in autophagosome precursor (Preautophagozomal structure; PAS) construction. ATG9 is only one membrane protein having a transmembrane domain among the ATG proteins and is localized in autophagosome precursors (Cell, 2010, 140 (3): 313-326).

Examples of the molecules involved in phagophore growth or autophagosome formation include ATG5, ATG7, ATG10, ATG12, ATG16 (or ATG16L1), ATG18 (or WIPI1-4), ATG3, ATG4 (or ATG4A-D), ATG8 (LC3, GATE-16, or GABARAP), and p62. Among these, ATG5, ATG7, ATG10, ATG12, and ATG16 form the Atg12-conjugation system. This system is present outside the phagophore and is considered to be important for phagophore extension. LC3, ATG3, ATG4, and ATG7 form the LC3/Atg8-conjugation system. This system is considered to be involved in phagophore extension and membrane closure (Cell, 2010, 140 (3): 313-326). LC3 is a mammalian homolog of ATG8, and there are forms of LC3-I and LC3-II. LC3-II of these is formed by phosphatidylethanolamine covalently bound to cytoplasm-localized LC3-I and is thereby localized on autophagosome membranes. Further, WIPI1 and WIPI2, which are human homologs of ATG18, are involved also in the ATG12-LC3 ubiquitin-like conjugation systems and are localized on autophagosome membranes (Autophagy, 2016, 12 (1): 1-222). The amount of LC3-II is proportional to the number of autophagosomes and therefore is known as an indicator of autophagy. For example, the autophagic activity can be quantitatively evaluated by adding autophagy inhibitors such as hydroxychloroquine and protein degradation inhibitors such as E-64-D or pepstatin A, and measuring the accumulated amount of LC3-II protein. Alternatively, the autophagic activity can be evaluated by detecting the amount of LC3-II protein for each sample and combining another known autophagy marker such as p62 (Autophagy, 2007, 3 (6): 542-545). p62 is a protein localized in autophagosome formation sites and interacting with LC3, and has a function to recruit autophagy target proteins to autophagosomes. Further, p62 is known as an autophagy-selective substrate, as well as Nbr1 (Mol Cell, 2009, 33 (4): 505-516).

Examples of the molecules involved in fusion between autophagosomes and lysosomes include UVRAG (or VPS38) and RAB7. UVRAG is known as molecules which promote the process of fusion between autophagosomes and lysosomes.

The names of autophagy-related genes or molecules, and other genes and molecules disclosed in this specification are in accordance with the NCBI database (www.ncbi.nlm.nih.gov/).

In this specification, the autophagy-related genes or molecules can include their homologs. In this specification, a gene or protein having high sequence identity and equivalent functions, and preferably a common evolutionary origin, with another gene or protein is referred to as a "homolog" of the other gene or protein. For example, a gene which is 60% or more, preferably 80% or more, more preferably 90% or more, identical to an autophagy-related gene in the nucleotide sequence and which is configured to encode a protein involved in each step of the autophagy pathway such as phagophore generation, phagophore extension or growth, autophagosome formation, fusion between autophagosomes and lysosomes, autolysosome formation, and degradation of substances inside autolysosomes, and preferably which is derived from the same evolutionary origin, is a homolog of the autophagy-related gene. For example, proteins and mRNA encoded by the homolog of the autophagy-related gene are also homologs of the autophagy-related molecule.

In the present invention, at least one of the autophagy-related genes or molecules needs only to be used. In other words, the genes or molecules may be individually used, or any two or more of them may be used in combination. For example, the autophagy-related genes or molecules used in the present invention need only to be at least one of the autophagy-related genes or molecules selected from the group consisting of the autophagy-related genes listed above, and mRNA and proteins encoded by them. More specifically, in the present invention, the genes listed above or mRNA and proteins encoded by them may be individually used, any two or more of the genes may be used in combination, any two or more of mRNA encoded by the genes may be used in combination, any two or more of proteins encoded by the genes may be used in combination, or any two or more of the genes, mRNA, and proteins may be used in combination.

In this specification, the "autophagic activity" refers to the activity in each step of the autophagy pathway such as phagophore generation, phagophore extension or growth, autophagosome formation, fusion between autophagosomes and lysosomes, autolysosome formation, and degradation of substances inside autolysosomes. For example, the autophagic activity is reflected to the expression or activity of the autophagy-related genes or molecules and can be measured by detecting or determining the expression or activity thereof. The expression or activity of the autophagy-related genes or molecules can be measured by any analysis method generally used in this field. Examples of the gene expression analysis method include dot blotting, Northern blotting, RNase protection assay, reporter assay by luciferase or the like, RT-PCR, and DNA microarray. Examples of the method for analyzing or quantitating the expression or activity of proteins encoded by the genes include Western blotting, immunostaining, fluorescent staining, ELISA, and binding assay.

In this specification, the "prevention" means to prevent, inhibit, or delay the onset of diseases, symptoms, or conditions in individuals, or to reduce the risk of the onset of diseases, symptoms, or conditions in individuals. Further in this specification, the "amelioration" means to turn up diseases, symptoms, or conditions, to prevent, inhibit, or delay a deterioration in diseases, symptoms, or conditions, or to reverse, prevent, inhibit, or delay the progress of diseases, symptoms, or conditions in individual.

In this specification, the "uneven skin tone" means a condition which remarkably occurs in skin over joints (such as elbows, knees, finger joints, and ankles), and in which the saturation (brilliance) and brightness (lightness) of the skin decrease as compared with the peripheral skin, as a result of which the skin looks discolored. More specifically, the "uneven skin tone" in this specification means a condition where the brightness and saturation of the skin decrease as compared with the peripheral skin, due to skin dryness, thickening, and pigmentation, as a result of which the skin looks discolored, or ashy skin condition with pigmentation. Such uneven skin tone is particularly remarkably observed at joints of races with dark skin tone such as African races, Hispanics, Latinas, and descendants of them. In such races with dark skin tone, skin symptoms in which the skin surface dries and looks to be powder-dusted (ashy) are frequently observed, and such symptoms are often referred to as "ashy skin" or "ashiness" (Eur J Dermatol, 2003, 13: 574-578). The uneven skin tone or ashy skin at joints may be observed also in any other races with skin tones which are not so dark such as European Caucasoids and Asian people in some cases. However, the uneven skin tone and ashy skin in such races do not appear so remarkably in most cases.

The uneven skin tone targeted by the present invention is uneven skin tone at joints. Preferably, the uneven skin tone targeted by the present invention is uneven skin tone at joints of races with dark skin tone such as such as African races, Hispanics, Latinas, and their descendants. The uneven skin tone targeted by the present invention is ashy skin with pigmentation.

### (2. Amelioration in uneven skin tone

Described is a material which is useful for preventing or ameliorating uneven skin tone. According to the present invention, uneven skin tone (such as ashy skin with pigmentation) which is observed particularly in the skin over joints can be prevented or ameliorated.

As will be described later in this specification, it was found from the study of the inventors that significant correlations exist between skin tone parameters measured for skin over joints and skin barrier functions. Further, it was found that thickening with abnormal keratinocyte growth and differentiation, and excessive accumulation of melanin have occurred in the epidermis of the skin over joints in ashy skin condition with a decrease in saturation and brightness of the skin. These findings revealed that the abnormalities in keratinocytes, and skin dryness, thickening, and pigmentation which are caused by the abnormalities are pathological conditions of uneven skin tone at joints. Accordingly, normalizing the abnormalities in keratinocytes in the skin with uneven tone, ameliorating the skin dryness and thickening, and promoting the degradation and elimination of excessive melanin have been expected to contribute to ameliorating uneven skin tone.

It has been reported that autophagy is involved in melanin metabolism in keratinocytes (Patent Literature 1). Further, as will be described later in this specification, it was found from the study of the inventors that the autophagic activity in keratinocytes is low in the skin over joints in ashy skin condition with a decrease in saturation and brightness of the skin. Further, it was unexpectedly found that the autophagic activity in keratinocytes at joints correlates with not only skin tone but also the water content of the skin. Further, as will be described later, the thickness of the epidermis and the stratum corneum, and the epidermal melanin amount both decrease by restoring the reduced autophagic activity in keratinocytes in the skin with uneven tone. These results show that the activation of autophagy in keratinocytes can normalize the homeostasis of keratinocyte growth and differentiation and promote the degradation and elimination of excessive melanin.

As described above, pathological conditions of uneven skin tone, and a deep involvement of the keratinocyte autophagic activity in the pathological conditions were revealed. Enhancement of the autophagic activity in keratinocytes can prevent or ameliorate not only melanin accumulation in skin but also abnormalities in keratinocytes which lead to skin dryness and thickening, so as to be capable of preventing or ameliorating uneven skin tone, more specifically, uneven skin tone with skin dryness, thickening, and pigmentation typified by ashy skin with pigmentation.

That is, substances which enhance the autophagic activity are substances which can prevent or ameliorate uneven skin tone. When substances which enhance the autophagic activity are administered to keratinocytes, the keratinocytes are normalized to inhibit skin dryness, thickening, and pigmentation, so that uneven skin tone can be prevented or ameliorated. Further, the ameliorating actions of such substances on the uneven skin tone can be evaluated using the autophagy activating actions of the substances as indicators.

### (2-1. In vitro method for evaluating or selecting agent for preventing or ameliorating uneven skin tone

Accordingly, one aspect of the present invention relates to am in vitro method for evaluating or selecting an agent for preventing or ameliorating uneven skin tone. The method comprises: administering test substances to cells; measuring changes in the autophagic activity caused by the test substances in the cells; and selecting a test substance which has enhanced the autophagic activity.

The types of test substances may be natural or synthesized, and may be single substances, compositions, or mixtures. The administration form of the test substances to the cells can be any form depending on the test substances. For example, the test substances may be added into a culture medium in which the cells are cultured, or the test substances may be applied directly to the cells which are being cultured.

The cells to which the test substances are administered may be natural or genetically modified as long as they have autophagic activity. The cells are preferably cultured cells derived from mammals, more preferably cultured human cells. The cells are preferably cultured skin cells, more preferably cultured keratinocytes. As the cultured keratinocytes, keratinocytes collected from animal skin and cultured according to a conventional method may be used, but commercially available established cell lines (such as HaCaT cells which are a human epidermal keratinocyte line) can also be used. Suitable examples of keratinocytes include human keratinocytes, for example, normal human epidermal keratinocytes (NHEK). The race from which the human keratinocytes are derived is not specifically limited, but races with dark skin tone such as African races, Hispanics, Latinas, and their descendants are preferable. The skin from which the human keratinocytes are derived is not specifically limited, but skin over joint sites (such as elbows, knees, finger joints, and ankles) is preferable, and skin over sites with uneven tone or in ashy skin condition is more preferable.

The autophagic activity caused by the test substances can be measured, for example, by investigating the expression or activity of the autophagy-related genes or molecules in the cells to which the test substances have been administered or the amount of autophagy-related molecules in the cells. Alternatively, the autophagic activity can be measured by investigating the activities of autophagy inhibitory factors (such as Rubicon and mTORC1), target factors of autophagy inducers (such as mTOR; mammalian target of rapamycin and AMPK; AMP-activated protein kinase), or transcription factors which change the autophagic activity. Examples of the transcription factors which change the autophagic activity include transcription factors belonging to the TFE family which activates autophagy (such as TFE3 and TFEB), transcription factors belonging to the FOXO family which also activates autophagy (such as FOXO1 and FOXO3), and ZKSKAN3 which contrarily inhibits autophagy. The expression or activity of the genes or molecules, the amount of molecules in the cells, or the activity of each factor can be measured by any analysis method generally used in the field. Examples of the gene expression analysis method include dot blotting, Northern blotting, RNase protection assay, reporter assay by luciferase or the like, RT-PCR, and DNA microarray, but there is no limitation to these examples. Examples of the method for analyzing or quantitating the expression or activity of proteins encoded by genes include Western blotting, immunostaining, fluorescent staining, ELISA, and binding assay, but there is no limitation to these examples.

Alternatively, preferable examples of evaluation of autophagic activity include investigating the amount of molecules involved in phagophore generation, phagophore growth, or autophagosome formation in the cells, investigating the activities of autophagy inhibitory factor, investigating the activities of target factors of autophagy inducers, and investigating the activities of transcription factors which change the autophagic activity. Specifically, a technique called flux assay is preferably used (Mizushima and Yoshimori, Autophagy, 2007, 3: 542-545). The flux assay is a quantitative analysis technique in which cells are cultured with or without the coexistence of a lysosome inhibitor(s), and the accumulated amount of LC3-II protein following a lysosomal inhibitor(s) treatment is measured as autophagic activity. As a technique for measuring the activities of autophagy inhibitory factor (such as Rubicon), measurement of the gene expression or protein expression respectively by quantitative RT-PCR or Western blotting is preferably used, for example. As a technique for measuring the activities of target factors of autophagy inducers, a method of analyzing phosphorylation of mTOR protein itself by Western blotting and detecting phosphorylation of target proteins (such as p70S6 kinase) phosphorylated by mTOR by Western blotting are preferably used, for example, in the case where the target factor is mTOR. For example, in the case where the target factor is AMPK, detection of phosphorylated AMPK protein by Western blotting is preferably used. Preferable examples of the method for evaluating the activities of transcription factors which change the autophagic activity include detecting proteins by Western blotting, and quantitatively evaluating the transcription-activating capacity by reporter assay or binding assay.

Preferably, changes in the autophagic activity caused by the test substance can be measured by comparing the autophagic activity measured in the cells to which the test substance has been administered with the autophagic activity in the control cells (cells to which the test substance has not been administered). For example, measured values before and after the administration can be compared with each other by measuring the autophagic activity before and after the administration of the test substance. Alternatively, autophagic activities of the test substance-administered group and the non-administration group or the control substance-administered group are measured, for example, and the measured values can be compared between the administration group and the non-administration group, or between the administration group and the control substance-administered group. Alternatively, changes in the autophagic activity caused by the test substances can be measured by administering test substances with different concentrations to the cells to measure their autophagic activities and investigating the difference in measurement results depending on the concentrations of the test substances.

Test substances which have enhanced autophagic activity in the cells are selected as agents for preventing or ameliorating uneven skin tone, more specifically, uneven skin tone with skin dryness, thickness, and discoloration typified by ashy skin with pigmentation. The selected test substances may be subjected to an additional evaluation test, to select a more preferable substance as an agent for preventing or ameliorating uneven tone. In the additional evaluation test, for example, effects of the test substances can be evaluated by administering the test substances to an individual with uneven skin tone or an organ culture of skin with uneven tone and then measuring changes in uneven skin tone. As the individual with uneven skin tone, races with dark skin tone such as African races, Hispanics, Latinas, and their descendants are preferable. As the skin with uneven tone to be organ-cultured, skin of the races with dark skin tone is preferable. As the site of the individual with uneven skin tone to which the test substances are administered or the site from which the skin with uneven tone to be organ-cultured is sampled, skin over sites with uneven tone, such as joint sites (for example, elbows, knees, finger joints, and ankles) is preferable, and skin over sites with uneven skin tone accompanied by skin dryness, thickening, and pigmentation typified by ashy skin is more preferable. Changes in uneven skin tone can be measured based on the direct observation of skin appearance, the skin tone, the skin moisture content, or the like. The skin tone can be measured according to a conventional method based on skin tone parameters such as brightness (L*), redness (a*), yellowness (b*), saturation (C*), and pigmentation (ITA°). The skin moisture content and barrier function can be measured according to a conventional method, for example, based on the stratum corneum water content (Capacitance or Conductance) and the trans-epidermal water loss (TEWL), respectively.

### (2-2. Non-therapeutic method for preventing or ameliorating uneven skin tone

Another aspect of the present invention relates to a non-therapeutic method for preventing or ameliorating uneven skin tone. The method comprises enhancing autophagic activity in keratinocytes in skin in need of the prevention or amelioration of uneven skin tone, wherein the uneven skin tone is skin discoloration with skin dryness, thickening, and pigmentation, or ashy skin with pigmentation, wherein the uneven skin tone is uneven tone in skin over joints, and wherein the enhancement of the autophagic activity in keratinocytes in the skin comprises administering at least one selected from the group consisting of Melia toosendan, its extract, and toosendanin to the skin.

Examples of the skin in need of the prevention or amelioration of uneven skin tone include the skin of living animal bodies with uneven tone, the skin of living animal bodies at sites where uneven tone tends to occur, and the skin of living animal bodies in which prevention of uneven tone is desired. The skin is skin over joints (such as elbows, knees, finger joints, and ankles). Examples of the animals include humans or non-human mammals, where humans are preferable. The humans are preferably of races with dark skin tone such as African races, Hispanics, Latinas, and their descendants. The skin in need of the prevention or amelioration of uneven skin tone is preferably human skin over joints, more preferably human skin in ashy skin condition, further preferably human skin over joints of races with dark skin tone such as African races, Hispanics, Latinas, and their descendants, still further preferably human skin over joints of races with dark skin tone such as African races, Hispanics, Latinas, and their descendants in ashy skin condition with pigmentation.

As one example of the non-therapeutic method for enhancing autophagic activity in keratinocytes, there is a method of changing the expression or activity of the autophagy-related genes or molecules in the cells. For example, an increase in expression of autophagy-related genes or molecules involved in autophagy activation in keratinocytes enhances the autophagic activity. Further, inhibition of expression of autophagy-related genes or molecules involved in inhibition of autophagic activity in keratinocytes enhances the autophagic activity, for example.

The autophagy-related genes or molecules involved in autophagy activation or inhibition of activity can be identified by investigating the influence of the expression of the genes or molecules on the autophagic activity using a general method. For example, genes or molecules whose expression changes when autophagic activity in cells is increased or decreased by administering autophagy inducers or autophagy inhibitors are determined. Subsequently, whether the genes or molecules are involved in autophagy activation or activity inhibition can be determined by investigating changes in the autophagic activity when the genes or molecules are overexpressed or the expression is inhibited. Alternatively, in the case of known autophagy-related genes or molecules, whether the genes or molecules are involved in autophagy activation or activity inhibition can be determined by investigating changes in the autophagic activity when the genes or molecules are overexpressed or the expression is inhibited.

For example, the molecules involved in phagophore generation (such as ATG1 (or ULK1 or ULK2), ATG13, ATG17, ATG29, ATG31, ATG101, FIP200, VPS34, VPS15 (or p150), ATG6 (or VPS30 or Beclin1), ATG14, Ambra1, ATG2, ATG9, ATG18 (or WIPI1-4), DFCP1, and VMP1), the molecules involved in phagophore growth or autophagosome formation (such as ATG5, ATG7, ATG10, ATG12, ATG16 (or ATG16L1), ATG18 (or WIPI1-4), ATG3, ATG4 (or ATG4A-D), and ATG8 (or LC3, GATE-16, or GABARAP)), and the molecules involved in fusion between autophagosomes and lysosomes (such as UVRAG (or VPS38), and RAB7) are involved in autophagy activation. Enhancing the expression or activity of these genes or molecules can prevent or ameliorate uneven skin tone. Meanwhile, Rubicon, p62, and mTORC1 are, for example, involved in inhibition of autophagic activity. Accordingly, as another example of the method for enhancing the autophagic activity in keratinocytes, inhibiting the expression or activity of the aforementioned genes or molecules can prevent or ameliorate uneven skin tone.

The expression or activity of the autophagy-related genes or molecules can be changed by any method generally used in the field. For example, examples of the method for changing the expression of the autophagy-related genes or the like include transcriptional activation of target genes by specific promoters, gene transfer from outside using vectors, knockdown of genes involved in inhibition of autophagic activity by antisense oligonucleotides, siRNA, or the like, and application of any substance having the action of changing the expression of other genes. Among these, the application of any substance having the action of changing the expression of the genes is preferable.

Examples of the vectors used for gene transfer include various gene expression vectors in which the CMV promoter or the EF1α promoter is incorporated as vectors for cultured cells, and examples of the vectors for cultured cells and tissues include adenoviral vectors, retroviral vectors, and lentiviral vectors. Procedure of gene transfer using the vectors is well known to those skilled in the art. For example, the procedure of gene transfer to skin using lentiviral vectors is described in Gene Ther. 2007 Apr, 14 (8): 648-56. Further, kits for gene transfer using these vectors are commercially available. The aforementioned vectors in which the autophagy-related genes are incorporated are transferred to the cells, and thereby the autophagy-related genes are overexpressed in the cells.

The gene knockdown by siRNA is a technique for specifically inhibiting the expression of target genes by directing mRNA degradation in a sequence-specific manner based on the mechanism of RNA interference (RNAi). siRNA is also used for in-vivo gene knockdown of various organisms including mammals (see Song et al., Nature Medicine, 2003, 9: 347-351; McCaffery et al., Nature, 2002, 418: 38-39; Lewis et al., Nature Genetics, 2002, 32: 107-108; and Xia et al., Nature Biotech., 2002, 20: 1006-1010, for example). siRNA which is synthesized specifically to a target can be purchased, and kits for siRNA transfection are sold by many traders such as QUIAGEN and Takara Bio Inc.

Alternatively, the autophagic activity in keratinocytes can be enhanced by adjusting the activities of transcription factors involved in the control of autophagy-related gene expression. For example, activation of TFE3 and TFEB which are transcription factors belonging to the transcription factor TFE family, and FOXO1 and FOXO3 which are transcription factors belonging to the FOXO family enhances the expression of autophagy-related genes (Nature Reviews Molecular Cell Biology, 2014, 15: 65-74). The autophagic activity can be enhanced by activating these transcription factors. Meanwhile, the activation of transcription factor ZKSKAN3 inhibits the autophagic activity. The autophagic activity can be enhanced by inhibiting this transcription factor.

Examples of the method for changing the expression or activity of autophagy-related molecules include a method of changing the expression of genes which encode the molecules, a method of changing the expression of proteins, a method of changing the enzyme activity or the like of the molecules, a method of changing the interaction between the molecules and the target factors, and a method of changing the signal path on which the molecules act. Among these, the method of changing the expression of genes and the method of changing the expression of proteins are, for example, preferable.

Other examples of the method for enhancing autophagic activity in keratinocytes include administration of autophagy inducers to keratinocytes. Examples of the autophagy inducers include mTOR signal inhibitors typified by rapamycin and Torin 1, proteasome inhibitors such as fluspirilene, trifluoperazine, pimozide, nicardipine, niguldipine, loperamide, amiodarone, verapamil, minoxidil, clonidine, PP242, or MG-132, and AMPK activators such as AICAR and metformin.

Still other examples of the method for enhancing autophagic activity in keratinocytes include inhibition or activation of target factors of the aforementioned autophagy inducers. For example, the autophagic activity in cells can be enhanced by inhibiting mTOR which is the target of the aforementioned autophagy inducer, rapamycin. mTOR is a kind of protein kinases (serine-threonine kinases) involved in intracellular signal transduction and has the action of inhibiting autophagy induction (Nat Genet, 2004 Jun, 36 (6): 585-95). Further, AMPK inhibits mTORC1 activity and promotes autophagy by directly activating ATG1 (or ULK1 or ULK2) (J Cell Biochem, 2012, 113, 695-710).

Preferably, the aforementioned methods for enhancing autophagic activity are locally applied to the skin in need of the prevention or amelioration of uneven skin tone. Examples of the method of local application include injection, transdermal administration, or the like of antisense oligonucleotide, siRNA, promoters, vectors, autophagy inducers, or the like to the target skin.

The prevention or amelioration of uneven skin tone by the present invention is non-therapeutically performed for cosmetic purposes or aesthetic purposes. In this specification, the word "non-therapeutically" means a concept not including medical practice, that is, a concept not including methods of surgery, treatment, or diagnosis in humans, more specifically, a concept not including methods of performing surgery, treatment, or diagnosis in humans by doctors or people who received instructions from doctors.

### (2-3. Prevention or amelioration of uneven skin tone using Melia toosendan)

Preferable examples of the autophagy inducers which can be used for preventing or ameliorating uneven skin tone by the present invention include Melia toosendan or its extract. In the present invention, any portion (such as whole tree, whole grass, root, rhizome, trunk, branch, stem, leaf, bark, sap, resin, flower, fruit, and seed, or combination thereof) of Melia toosendan can be used, where fruit, bark, or combination thereof is preferably used. For example, senrenshi which is a crude drug obtained using the fruit of Melia toosendan as the original plant, or kurenpi which is a crude drug obtained using the bark thereof as the original plant can be used. The Melia toosendan used in the present invention may be a raw plant or may be a dried plant product. More preferably, the Melia toosendan used in the present invention is senrenshi.

The Melia toosendan extract used in the present invention can be obtained by extraction of the plant by a general method. The Melia toosendan extract can be juice extracted by pressing a dried plant product or a ground product thereof, steam distillate, crude extract using various extraction solvents, and a fraction of the extract obtained by purifying the crude extract by various chromatography methods such as partition or column chromatography. The raw plant can be subjected to extraction, as it is, but is preferably subjected to processes such as drying, chopping, and grinding for enhancing the extraction efficiency. Further, in the present invention, any one of the aforementioned examples such as extract, steam distillate, and a compressed product may be used alone, or two or more of them may be used in combination. Preferably, the Melia toosendan extract is extract obtained from a dried product of Melia toosendan or a ground product thereof using an extraction solvent.

As the extraction solvent, any one of polar solvents and non-polar solvents can be used, or these solvents can be used as a mixture. Examples thereof include water; alcohols such as methanol, ethanol, propanol, and butanol; polyhydric alcohols such as ethylene glycol, propylene glycol, and butylene glycol; ketones such as acetone and methyl ethyl ketone; esters such as methyl acetate and ethyl acetate; linear and cyclic ethers such as tetrahydrofuran and diethyl ether; polyethers such as polyethylene glycol; hydrocarbon halides such as dichloromethane, chloroform, and carbon tetrachloride; hydrocarbons such as hexane, cyclohexane, and petroleum ether; aromatic hydrocarbons such as benzene and toluene; pyridines; supercritical carbon dioxide; and fats and oils, waxes and other oils. Alternatively, a mixture obtained by combining two or more of the aforementioned solvents can be used as the extraction solvent. Among these, water, alcohols, water-alcohol mixtures, propylene glycol, butylene glycol, or water-polyhydric alcohol mixtures are preferably used, and an ethanol aqueous solution or a 1,3-butylene glycol aqueous solution is more preferably used. The concentration of alcohols and polyhydric alcohols in the water-alcohol mixtures and the water-polyhydric alcohol mixtures is preferably 50 (v/v)% or more, more preferably 60 (v/v)% or more, further preferably 70 (v/v)% or more, and is preferably 98 (v/v)% or less, more preferably 95 (v/v)% or less, further preferably 90 (v/v)% or less. The concentration range is preferably from 50 to 98 (v/v)%, more preferably from 60 to 95 (v/v)%, further preferably from 70 to 90 (v/v)%.

The extraction conditions for obtaining the extract can differ depending on the solvent used and are not particularly limited. For example, in the case of extraction using water, alcohols, water-alcohol mixtures, propylene glycol, butylene glycol, or water-polyhydric alcohol mixtures, immersion or heating reflux preferably using from 1 to 50 parts by volume of a solvent with respect to 1 part by mass of plants, preferably at a temperature of from 3 to 100°C, more preferably from 20 to 80°C, preferably for from 1 hour to several weeks, more preferably from one day to 30 days is preferable. Further, in order to increase the extraction efficiency, stirring, homogenization in a solvent, or the like may be performed in combination.

The obtained extract may be used as it is, but it can be used by further separating a fraction with higher activity by a suitable separation method such as gel filtration, chromatography, and precision distillation. The Melia toosendan extract used in the present invention includes various extracts thus obtained, diluted solutions thereof, concentrated solutions thereof, purified products thereof, or dried powders thereof.

Alternatively, preferable examples of the autophagy inducers which can be used for preventing or ameliorating uneven skin tone by the present invention include toosendanin (CAS 58812-37-6) which is the main structural component of the Melia toosendan extract. As will be shown later in Examples, it was found that senrenshi extract and toosendanin have an autophagy-activating action, and uneven skin tone is ameliorated by applying the senrenshi extract.

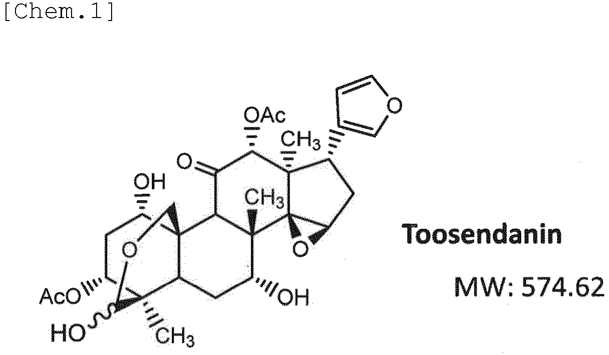

Accordingly, another aspect of the present invention relates to use of Melia toosendan, its extract, and toosendanin as a cosmetic composition for preventing or ameliorating uneven skin tone.

Further, the present invention provides a non-therapeutic method for preventing or ameliorating uneven skin tone, the method comprising: administering at least one selected from the group consisting of Melia toosendan, its extract, and toosendanin to skin in need of the prevention or amelioration of uneven skin tone, wherein the uneven skin tone is skin discoloration with skin dryness, thickening, and pigmentation, or ashy skin with pigmentation, wherein the uneven skin tone is uneven tone in skin over joints. In other words, in the non-therapeutic method for preventing or ameliorating uneven skin tone according to the present invention, the enhancement of autophagic activity in keratinocytes in the skin preferably includes administering at least one selected from the group consisting of Melia toosendan, its extract, and toosendanin to the skin.

Preferably, in the present invention, the at least one selected from the group consisting of Melia toosendan, its extract, and toosendanin is transdermally administered. Preferably, the agent for preventing or ameliorating uneven skin tone is a skin external preparation.

In one embodiment, the agent for preventing or ameliorating uneven skin tone can be substantially composed of at least one selected from the group consisting of Melia toosendan, its extract, and toosendanin. In another embodiment, the agent for preventing or ameliorating uneven skin tone can be a composition containing at least one selected from the group consisting of Melia toosendan, its extract, and toosendanin. Examples of the composition include a cosmetic composition, which will be described below.

The use of at least one selected from the group consisting of Melia toosendan, its extract, and toosendanin according to the present invention is non-therapeutically performed for cosmetic purposes or aesthetic purposes. Preferably, the agent for preventing or ameliorating uneven skin tone is a cosmetic composition for preventing or ameliorating uneven skin tone. The use of at least one selected from the group consisting of Melia toosendan, its extract, and toosendanin of the present invention is use as a cosmetic composition for preventing or ameliorating uneven skin tone. In the cosmetic composition, the at least one selected from the group consisting of Melia toosendan, its extract, and toosendanin is used as an active ingredient for imparting a function of preventing or ameliorating uneven skin tone to the composition.

The form of the cosmetic composition is not particularly limited and can be in any form including solutions, milky lotions, powders, water-oil two-layer systems, water-oil-powder three-layer systems, gels, solid such as tablets, aerosol, mist, capsule, and sheet forms. Further, the product form of the cosmetic composition is also arbitrary, and examples thereof include skin care cosmetics such as lotions, serums, packs, milky lotions, and creams, makeup cosmetics such as foundations, and body cleaning agents such as soaps and body soaps. Preferably, the cosmetic composition is in the form of a skin external composition (or a composition for transdermal administration).

The cosmetic composition can be produced using a conventional method by mixing other ingredients which are conventionally used for cosmetics, quasi drugs, and pharmaceutical products, for example, active components such as humectants, whitening agents, and antiphlogistics, powders, fats and oils, fatty acids, alcohols, esters, silicones, surfactants, water-soluble polymers, thickeners, coating agents, ultraviolet absorbers, sequestrants, pH adjusters, antioxidants, preservatives, perfumes, water, and other additives, as required. The cosmetic composition provided by the present invention may belong to either cosmetics or quasi drugs according to the Japan pharmaceutical Affairs Act.

The content of the active ingredient (at least one selected from the group consisting of Melia toosendan, its extract, and toosendanin) of the present invention in the cosmetic composition is not specifically limited. In the case where the active ingredient is Melia toosendan extract, the content can be preferably 0.0000001 % by mass or more, more preferably 0.000001 % by mass or more, further preferably 0.00001 % by mass or more, further preferably 0.0001 % by mass or more, further preferably 0.001 % by mass or more, further preferably 0.01 % by mass or more, and preferably 1 % by mass or less, more preferably 0.5 % by mass or less, further preferably 0.1 % by mass or less, further preferably 0.05 % by mass or less, in terms of a dried product. Alternatively, the content of the Melia toosendan extract in the cosmetic composition can be preferably from 0.0000001 to 1 % by mass, 0.000001 to 1 % by mass, 0.00001 to 1 % by mass, 0.0001 to 1 % by mass, from 0.001 to 1 % by mass, from 0.01 to 1 % by mass, from 0.0000001 to 0.5 % by mass, from 0.000001 to 0.5 % by mass, from 0.00001 to 0.5 % by mass, from 0.0001 to 0.5 % by mass, from 0.001 to 0.5 % by mass, from 0.01 to 0.5 % by mass, from 0.0000001 to 0.1 % by mass, from 0.000001 to 0.1 % by mass, from 0.00001 to 0.1 % by mass, from 0.0001 to 0.1 % by mass, from 0.001 to 0.1 % by mass, from 0.01 to 0.1 % by mass, or from 0.01 to 0.05 % by mass, in terms of a dried product.

In the case where the active ingredient used according to the present invention is toosendanin, the content thereof in a cosmetic composition can be preferably 0.00000001 % by mass or more, more preferably 0.0000001 % by mass or more, further preferably 0.000001 % by mass or more, further preferably 0.00001 % by mass or more, and preferably 0.1 % by mass or less, more preferably 0.01 % by mass or less, further preferably 0.001 % by mass or less, further preferably 0.00005 % by mass or less. Alternatively, the content of toosendanin in the cosmetic composition can be preferably from 0.00000001 to 0.1 % by mass, more preferably from 0.0000001 to 0.01 % by mass, further preferably from 0.000001 to 0.001 % by mass, further preferably from 0.00001 to 0.00005 % by mass.

In the present invention, the dose of the active ingredient (at least one selected from the group consisting of Melia toosendan, its extract, and toosendanin) is not specifically limited, as long as it is an amount which allows the preventing or ameliorating effect on uneven skin tone to be obtained. The dose and administration plan of the active ingredient can be appropriately determined by those skilled in the art according to the race, body weight, gender, age, condition, or other factors of the target individual. For example, for an adult (60 kg) per day, the dose of Melia toosendan extract (in terms of a dried product) is preferably 0.001 mg or more and 1000 mg or less, or the dose of toosendanin is preferably 0.1 ng or more and 100 mg or less. The dose range of Melia toosendan extract (in terms of a dried product) per day is preferably from 0.001 to 1000 mg or from 0.001 to 100 mg/60 kg (body weight), and the dose range of toosendanin is preferably from 0.1ng to 100 mg or from 0.1 ng to 1 mg, for example. In the present invention, the aforementioned dose may be administered once to several times a day.

### (3. Exemplary Embodiments)

The present invention includes an in vitro method for evaluating or selecting an agent for preventing or ameliorating uneven skin tone, the method comprising: administering test substances to cells; measuring changes in autophagic activity caused by the test substances in the cells; and selecting a test substance which has enhanced the autophagic activity.

The uneven skin tone may be skin discoloration with skin dryness, thickening, and pigmentation, or ashy skin with pigmentation.

The uneven skin tone may be uneven tone in skin over joints (such as elbows, knees, finger joints, and ankles). The cells are preferably cultured cells, more preferably cultured keratinocytes, further preferably cultured human keratinocytes.

The cultured human keratinocytes may be derived from African races, Hispanics, Latinas, and their descendants. The cultured human keratinocytes are preferably derived from skin over joints (such as elbows, knees, finger joints, and ankles), more preferably derived from skin with uneven tone or in ashy skin condition.

The autophagic activity is preferably selected from the group consisting of phagophore generation, phagophore growth, autophagosome formation, fusion between autophagosomes and lysosomes, autolysosome formation, and degradation of substances inside autolysosomes, more preferably selected from the group consisting of phagophore generation, phagophore growth, and autophagosome formation.

The autophagic activity is preferably selected from the group consisting of the amount of molecules involved in phagophore generation, phagophore growth, or autophagosome formation in the cells, activities of autophagy inhibitory factor, activities of target factors of autophagy inducers, and activities of transcription factors which change the autophagic activity.

The autophagic activity is preferably the expression or activity of autophagy-related genes or molecules.

The measurement of changes in the autophagic activity is preferably measurement of changes in the activities of autophagy inhibitory factor, target factors of autophagy inducers, or transcription factors which change the autophagic activity, more preferably measurement of changes in the activity of Rubicon, mTORC1, mTOR, AMPK, ZKSKAN3, TFE3, TFEB, FOXO1, or FOXO3.

The measurement of changes in the autophagic activity is preferably any one of (a) to (d) below, more preferably any one of (a), (c), and (d) below, further preferably (d) below: (a) measuring an accumulated amount of LC3-II protein following a lysosomal inhibitor treatment as the autophagic activity; (b) measuring the amount of gene expression or protein expression of Rubicon as the autophagic activity; (c) measuring phosphorylation of mTOR protein or phosphorylation of a target protein phosphorylated by mTOR as the autophagic activity; and (d) measuring phosphorylated AMPK protein as the autophagic activity.

The method may include comparing the autophagic activity measured in the cells to which the test substances have been administered with the autophagic activity in control cells.

The method may include administering the selected test substance to an individual with uneven skin tone or an organ culture of skin with uneven tone; and measuring changes in the uneven skin tone.

The individual with uneven skin tone is preferably an individual of a race with dark skin tone selected from African races, Hispanics, Latinas, and their descendants. The test substance is preferably administered to the skin with uneven tone of the individual, more preferably administered to the skin with uneven tone over joint sites (such as elbows, knees, finger joints, and ankles) of the individual, further preferably administered to the skin in ashy skin condition of the individual.

The organ culture of the skin is preferably an organ culture of skin derived from a race with dark skin tone selected from African races, Hispanics, Latinas, and their descendants.

The organ-cultured skin is preferably skin over sites with uneven tone, more preferably skin with uneven tone at joints, further preferably skin in ashy skin condition.

The present invention further includes a non-therapeutic method for preventing or ameliorating uneven skin tone, the method comprising: enhancing autophagic activity in keratinocytes in skin in need thereof, wherein the uneven skin tone is skin discoloration with skin dryness, thickening, and pigmentation, or ashy skin with pigmentation, wherein the uneven skin tone is uneven tone in skin over joints, and wherein the enhancement of the autophagic activity in keratinocytes in the skin comprises administering at least one selected from the group consisting of Melia toosendan, its extract, and toosendanin to the skin.

The autophagic activity is preferably selected from the group consisting of phagophore generation, phagophore growth, autophagosome formation, fusion between autophagosomes and lysosomes, autolysosome formation, and degradation of substances inside autolysosomes, more preferably selected from the group consisting of phagophore generation, phagophore growth, and autophagosome formation.

The autophagic activity is preferably the expression or activity of autophagy-related genes or molecules.

The enhancement of changes in the autophagic activity preferably includes inhibition or activation of target factors of autophagy inducers, activation of transcription factors which enhance the expression of autophagy-related genes, or inhibition of transcription factors which inhibit the autophagic activity.

The enhancement of the autophagic activity in keratinocytes in the skin preferably comprises administering an autophagy inducer to the skin.

The enhancement of the autophagic activity in keratinocytes in the skin may comprise administering at least one selected from the group consisting of Melia toosendan, its extract, and toosendanin to the skin.

The present invention further includes a non-therapeutic method for preventing or ameliorating uneven skin tone, the method comprising: administering at least one selected from the group consisting of Melia toosendan, its extract, and toosendanin to skin in need thereof, wherein the uneven skin tone is skin discoloration with skin dryness, thickening, and pigmentation, or ashy skin with pigmentation, wherein the uneven skin tone is uneven tone in skin over joints.

The administration is preferably transdermal administration.

The skin is preferably skin of a race with dark skin tone selected from African races, Hispanics, Latinas, and their descendants, more preferably human skin over joints, further preferably human skin over joints of the race with dark skin tone, further preferably skin in ashy skin condition.

The present invention further includes the use of at least one selected from the group consisting of Melia toosendan, its extract, and toosendanin, as a cosmetic composition for preventing or ameliorating uneven skin tone, wherein the uneven skin tone is skin discoloration with skin dryness, thickening, and pigmentation, or ashy skin with pigmentation, wherein the uneven skin tone is uneven tone in skin over joints.

The cosmetic composition is preferably transdermally administered.

The cosmetic composition preferably comprises the Melia toosendan extract in an amount of from 0.0000001 to 1 % by mass, from 0.000001 to 1 % by mass, from 0.00001 to 1 % by mass, from 0.0001 to 1 % by mass, from 0.001 to 1 % by mass, from 0.01 to 1 % by mass, from 0.0000001 to 0.5 % by mass, from 0.000001 to 0.5 % by mass, from 0.00001 to 0.5 % by mass, from 0.0001 to 0.5 % by mass, from 0.001 to 0.5 % by mass, from 0.01 to 0.5 % by mass, from 0.0000001 to 0.1 % by mass, from 0.000001 to 0.1 % by mass, from 0.00001 to 0.1 % by mass, from 0.0001 to 0.1 % by mass, from 0.001 to 0.1 % by mass, or from 0.01 to 0.1 % by mass, more preferably from 0.01 to 0.05 % by mass, in terms of a dried product.

The cosmetic composition preferably comprises the toosendanin in an amount of from 0.00000001 to 0.1 % by mass, more preferably from 0.0000001 to 0.01 % by mass, further preferably from 0.000001 to 0.001 % by mass, further preferably from 0.00001 to 0.0001 % by mass, further preferably from 0.00001 to 0.00005 % by mass.

### [Examples]

### (4. Examples)

Examples are shown below, and the present invention will be described more specifically.

### [Example 1]

In order to reveal the reality of uneven skin tone, non-invasive measurements and tissue analyses were performed on skin biopsy. Test subjects were twelve African American (AA) women and three Caucasian women in their from 30s to 50s.

### (1) Non-invasive measurements

The skin at a healthy site (upper arm) without uneven tone and the skin at a pigmented site (elbow) of the test subjects were photographed and non-invasively measured. The skins were photographed using i-scope (Moritex). The skin tone was measured using Minolta CR-300 Chromameter, to evaluate the brightness (L*), redness (a*), and yellowness (b*) of the skins, and the saturation (C*) and pigmentation (ITA°) of the skins. As indicators of the stratum corneum functions of the skins, the stratum corneum water content (Capacitance) and trans-epidermal water loss (TEWL) were measured. TEWL is an indicator of a skin barrier function, and a higher TEWL indicates that the skin barrier function is reduced. Capacitance was measured using Corneometer CM 825 (Courage + Khazaka, Cologne, Germany). TEWL was measured using TEWA meter 300 (Courage + Khazaka, Cologne, Germany). The measurements were performed in an environment at room temperature of 21°C ± 2°C and a humidity of 45% ± 15% with an acclimation time of 15 minutes.

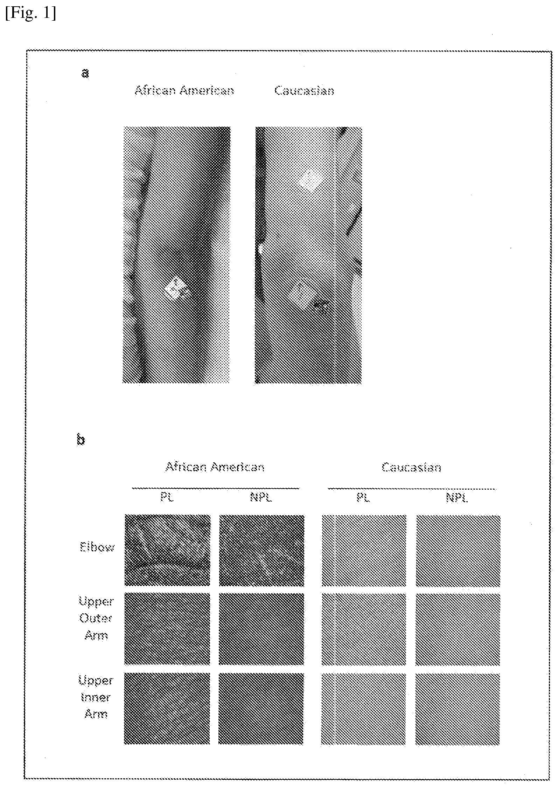

In the skin at the elbow site of AA, pigmentation was observed as compared with the skin at the outer and inner upper arm. Such pigmentation was not observed in Caucasians (Figures 1a and 1b). Further, in the skin at the elbow site of AA, a dry condition called ashy skin in which the skin appeared powder-dusted in ash color were remarkably observed, particularly, in photographs without polarization (Non-polarized; NPL) (Figure 1b).

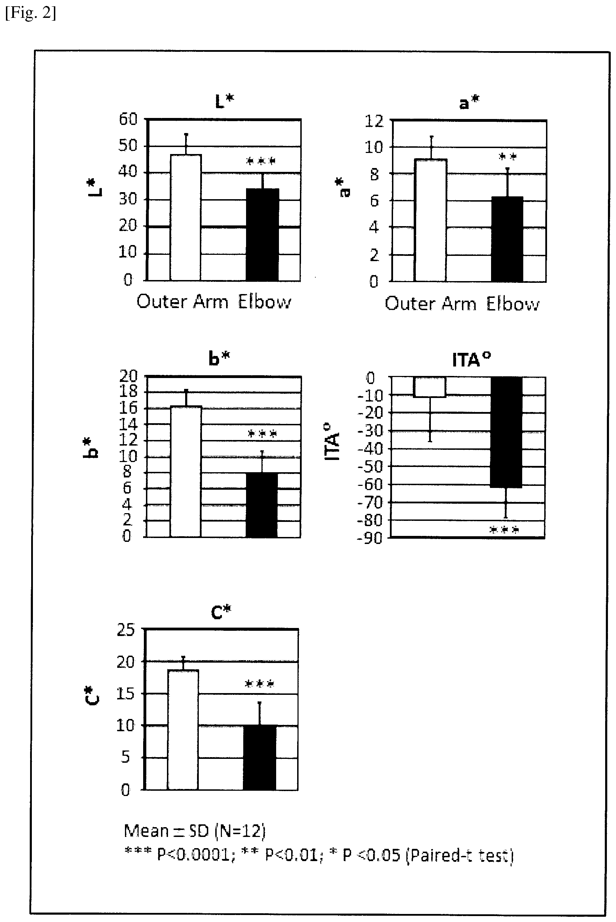

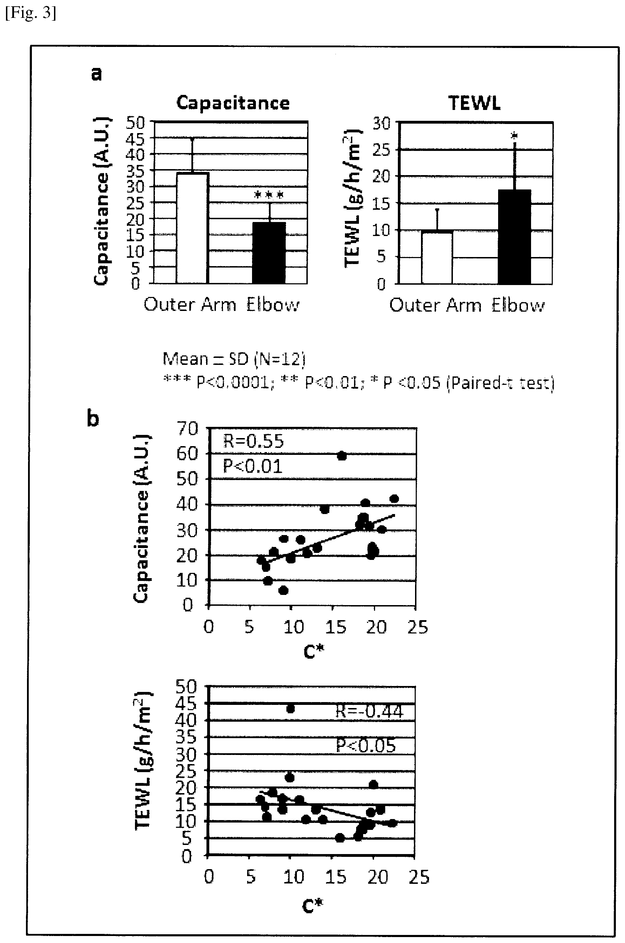

As a result of the skin tone measurements, all the five indicators of the brightness (L*), redness (a*), and yellowness (b*) of the skin, and the saturation (C*) and pigmentation (ITA°) of the skin at the elbow site of AA were statistically significantly low, as compared with those at the upper outer arm site of AA (Figure 2, paired t-test, p < 0.05). Further, in the skin at the elbow site of AA, TEWL was statistically significantly high, while the stratum corneum water content was statistically significantly low, as compared with those at the upper arm site (Figure 3a, paired t-test, p < 0.05). Further, as typified by the skin saturation (C*), statistically significant correlations were recognized between the parameters of the skin tone and the indicators of the stratum corneum functions (Figure 3b, Regression analysis, p < 0.05). Accordingly, it was found that the uneven skin tone which is characteristic of AA at joint sites was accompanied by a dry skin surface and a barrier function decrease together with changes in parameters of skin tone.

### (2) Tissue analyses on skin biopsy

From the outer and inner upper arm and elbow sites of the test subjects, skin tissues were collected through punch biopsy by a dermatologist. After the collection, frozen samples were rapidly produced, and immunohistochemical analyses were performed using their section.

The technique of immunohistochemical analyses will be described. The sections were immersed in acetone or a 4% PFA/PBS solution for 10 minutes to be fixed. The fixed sections were washed with PBS-T (0.05% Tween-20/PBS) for 5 minutes 3 times in total and were blocked with a 1% BSA/PBS solution for 30 minutes. Subsequently, after the sections were subjected to overnight treatment at 4°C with a primary antibody and were washed with PBS for 5 minutes 3 times in total, a secondary antibody (Jackson ImmunoResearch, 1:100 or 1:200) fluorescent labeled with FITC, RR-X, or Cy3 was added dropwise thereto, followed by treatment at 37°C for 30 minutes. Thereafter, the sections were washed with PBS-T for 5 minutes 3 times in total and with PBS for 5 minutes once, mounted by aqueous encapsulant containing a nuclear-staining agent (H-1200: Vector Laboratories). Refer to Table 1 for the type of primary antibody, the tissue fixation conditions, and the concentration used.

**[Table 1]**

| Primary antibody used for immunohistochemical analyses | | | |
|---|---|---|---|
| Antibody | Vendor, Catalog No. | Fixation solution | Dilution |
| Ki67 | DAKO, M7240 | 4%PFA/PBS | 1: 100 |
| Filaggrin | Abcam, ab3137 | Cold-Acetone | 1: 500 |
| Loricrin | BioLegend, 905101 | Cold-Acetone | 1: 1,000 |
| Transglutaminase-1 (TGM1) | Novus, NB100-1844 | Cold-Acetone | 1: 400 |
| Pmel17 | DAKO, M0634 | 4%PFA/PBS | 1: 40 |

Meanwhile, the sections were subjected to melanin staining (Melanin Stain), separately. The sections were immersed in a 4% PFA/PBS solution for 10 minutes to be fixed. The fixed sections were washed with distilled water (DDW) for 5 minutes 3 times in total, and thereafter a Fontana-ammonia silver solution (Muto) was added dropwise thereto, followed by treatment at room temperature for 30 minutes. Then, the sections were washed with DDW for 5 minutes 3 times in total, and a Kernechtrot solution (Muto) was added dropwise thereto, followed by treatment for 3 minutes, for nuclear staining. The excess staining solution was washed away with DDW, followed by dehydration, clearing, and mounting with Malinol (Muto).

The produced preparations were subjected to tissue observation and photographing using an inverted microscope (AxioVision, manufactured by Carl Zeiss Vision International GmbH) or a confocal laser microscope (LSM710, manufactured by Carl Zeiss Vision International GmbH). The captured images were analyzed using an image analysis software, and the expression levels of Pmel17, which is a marker of melanocyte growth and activation, cell proliferation marker Ki67, Filaggrin (FLG), Loricrin (LOR), and Transglutaminase 1 (TGM1), which are keratinization-related factors, were quantitated. Further, using RGB Profile Plot, which is an image analysis software, the fluorescence intensity distribution in the skin depth direction was investigated. The image analysis software used was "Image J" (available from imagej.nih.gov/ij/).

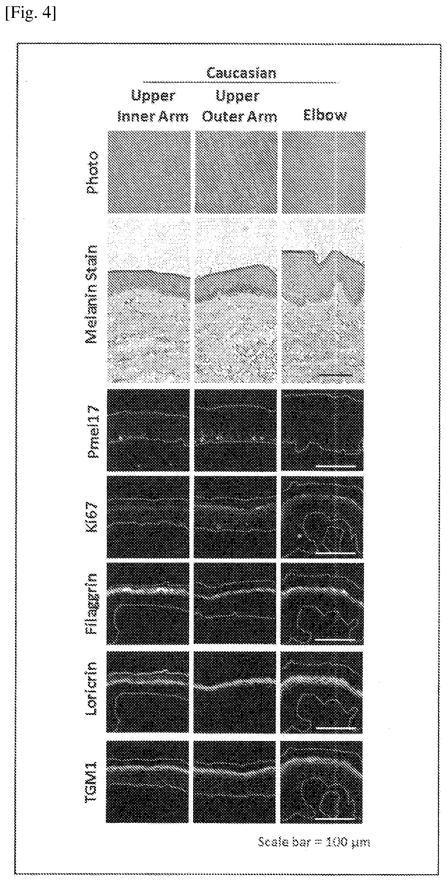

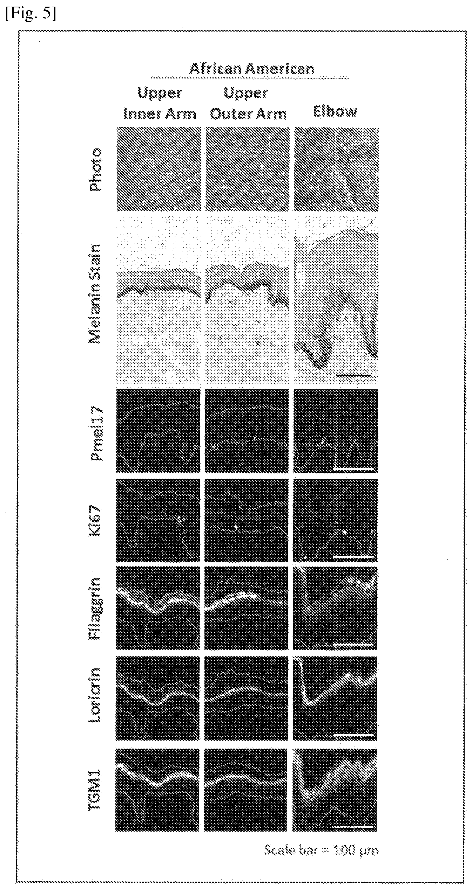

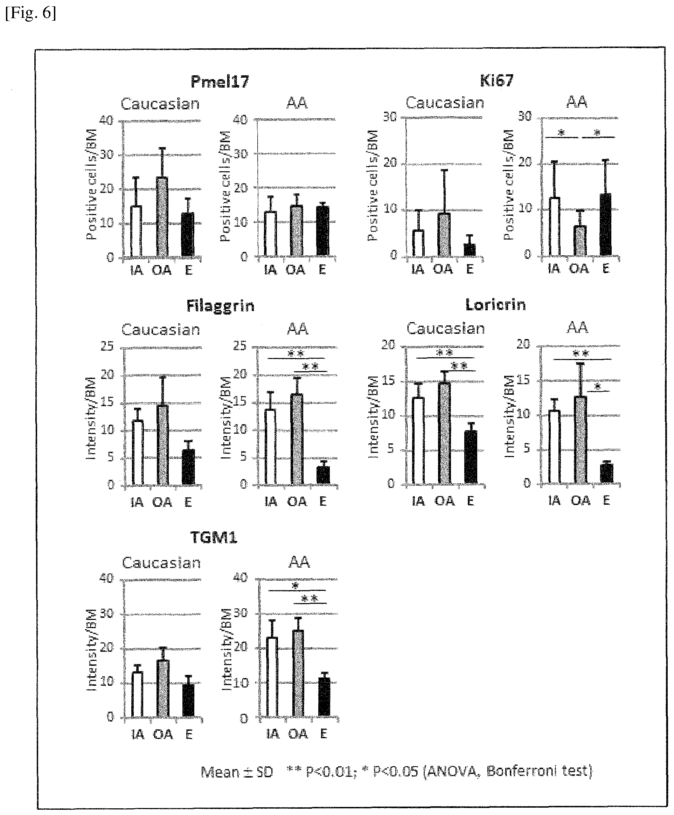

In Caucasian skins, no clear morphological difference was observed between the elbow site and the upper arm site. Meanwhile, significant morphological differences such as stratum corneum deposition accompanied by melanin deposition, and epidermal thickening were observed in the skin at the elbow site of AA, as compared with the skin at the upper arm site (Photo and Melanin Stain images in Figures 4 and 5). As shown in immunohistochemically stained images in Figures 4 and 5 and their quantitative analysis results (Figure 6), no increase in Pmel17 was observed at the elbow sites of both Caucasian and AA. Meanwhile, it was recognized that the number of Ki67 positive cells tended to be larger in the epidermis at the elbow site of AA as compared with that in the epidermis at the upper outer arm which is the vicinity of the elbow site. Further, it was observed by RGB Profile Plot that disturbance in localization of Filaggrin (FLG) and Loricrin (LOR), which are keratinized proteins normally selectively localized from the epidermal granular layer to the stratum corneum, occurred at the elbow site of AA. Also for TGM1, which is another keratinization-related factor, diffusion and non-uniform localization was observed at the elbow site of AA. As a result of quantitative analyses, FLG, LOR, and TGM1 decreased at the elbow site of AA as compared with those at the upper arm site thereof. These results suggested that, at the site of AA with uneven skin tone, epidermal thickening and excessive accumulation of melanin in the epidermis were caused together with abnormalities in keratinocyte growth and differentiation, while melanin synthesis by melanocytes remained unchanged.

### [Example 2]

It has been reported that autophagy is involved in melanin (melanosome) degradation in keratinocytes (Patent Literature 1). Immunohistochemical analyses were performed, focusing on autophagy in keratinocytes, based on the fact that excessive accumulation of melanin in epidermal tissues was observed in the skin with uneven tone. Using the skin samples collected in Example 1 (2) for the analyses, LC3, which is a protein localized in autophagosome and known as an autophagy marker, p62, which is a protein selectively degraded by autophagy, and ATG5-ATG12 complex, ATG16L1, and ATG9A, which are proteins localized in phagophore in the autophagosome membrane formation of the autophagy process prior to LC3, were stained. From the immunostaining images, the expression levels of autophagy-related molecules were quantified by the same procedure as in Example 1 (2). Refer to Table 2 for the type of primary antibody, the tissue fixation conditions, and the concentration used.

**[Table 2]**

| Primary antibody used for immunohistochemical analyses | | | |
|---|---|---|---|
| Antibody | Vendor, Catalog No. | Fixation solution | Dilution |
| LC3 | Cell signaling, 2775S | 4%PFA/PBS | 1: 100 |
| ATG5-ATG12 | Sigma, A0856 | 4%PFA/PBS | 1: 400 |
| ATG9A | MBL, PD042 | 4%PFA/PBS | 1: 400 |
| ATG16L1 | LifeSpan, LS-B8297 | 4%PFA/PBS | 1: 300 |
| p62/SQSTM1 (p62) | MBL, M162-3 | 4%PFA/PBS | 1: 40 |

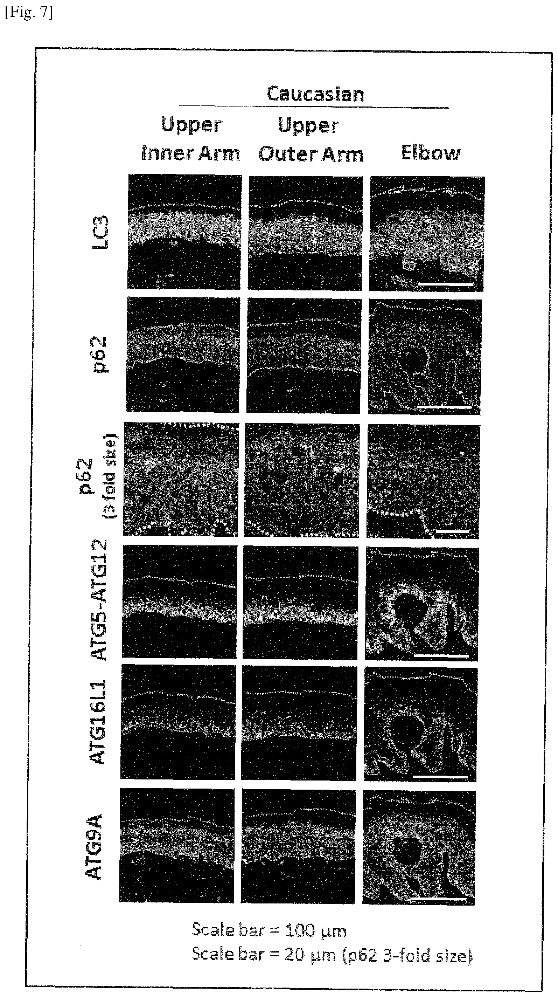

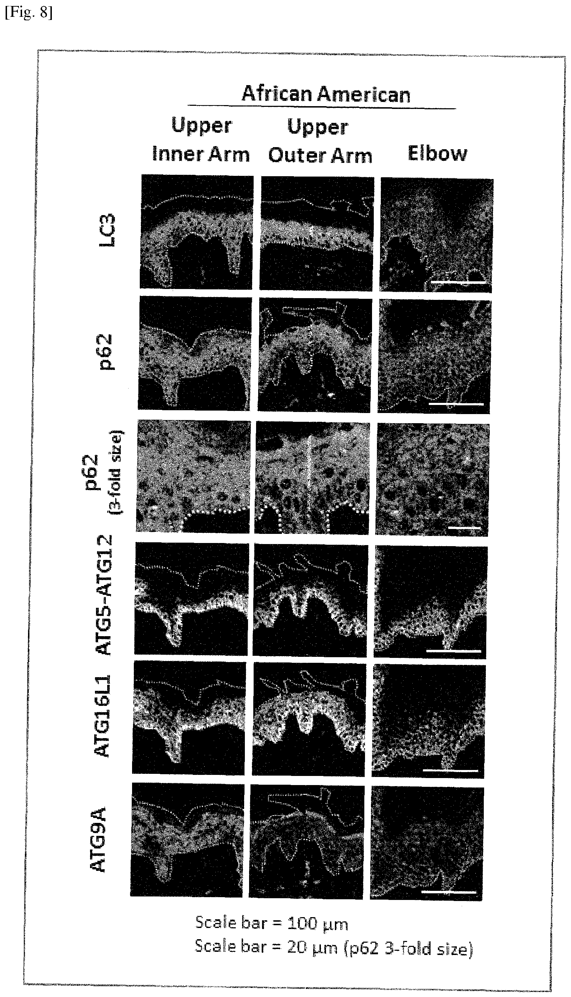

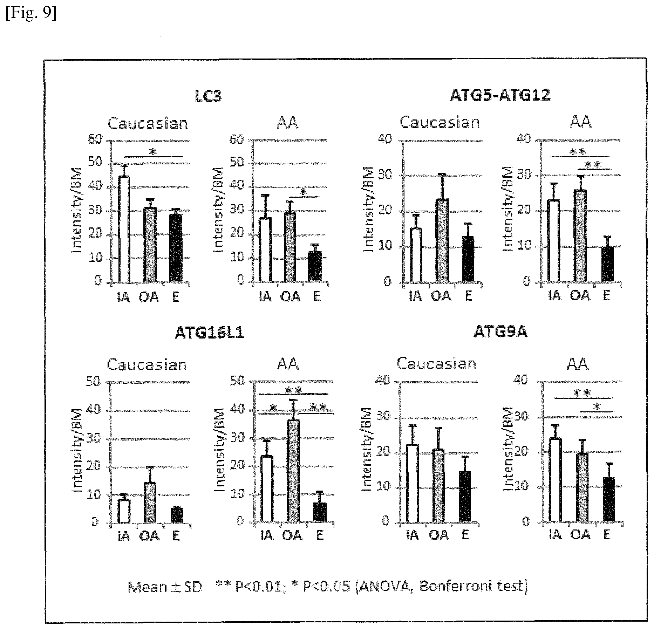

Figure 7 and 8 show the immunostaining images. LC3 was remarkably decreased in the epidermis at the elbow site of AA as compared with that at other sites. Meanwhile, p62 was emerged in the epidermis at the elbow site of AA. These facts suggested that autophagy was inhibited in the epidermis at the elbow site of AA. Localization of ATG5-ATG12, ATG16L1, and ATG9A was observed in Caucasians with no uneven skin tone and AA at the upper arm site from the basal layer of the epidermis around the spinous layer, but attenuation in fluorescence intensity was observed at the elbow site of AA, where a decrease in expression was suggested. As shown in the quantitative analysis results in Figure 9, a significant difference in expression of autophagy-related molecules (LC3, ATG5-ATG12, ATG16L1, ATG9A) in AA was recognized between the elbow site and the healthy site (the inner or outer upper arm). Accordingly, it was suggested that the melanosome degradation ability by autophagy possibly decreased in keratinocytes at the joint sites of AA with uneven skin tone.

### [Example 3]

Since a decrease in autophagy in the skin at the elbow site of AA was suggested by the histochemical analyses, LC3-II Flux assay for quantifying the autophagic activity was performed. The amount of LC3-II which is molecules localized in autophagosome is a quantitative indicator showing the original level of the autophagic activity of the cells. In the assay, autophagosome degradation was inhibited by applying an autophagy inhibitor, Hydroxychloroquine (HCQ) to the cells, and the amount of LC3-II accumulated in the cells was then measured. A ratio of the amount of LC3-II measured in the presence of HCQ with respect to the amount in the absence of HCQ was determined, to quantify the autophagic activity. The quantified autophagic activity at the upper outer arm was compared with that at the elbow site.

From two points at the upper outer arm site and the elbow site of five AA women, skin tissues were collected through punch biopsy by a dermatologist. Before the collection, the skin tone, the stratum corneum water content, and the trans-epidermal water loss at the tissue collection sites had been measured, in the same manner as in Example 1. The collected tissues were halved with a scalpel and were cultured in the presence or absence of a 20- *µ*M HCQ for 48 hours. In the tissue culture, a culture medium obtained by adding 10v/v% Fetal Bovine Serum (FBS) and antibiotics (Antibiotic-Antimycotic) to Advanced-DMEM was used (both were from Life Technologies). From the skin tissues after the culture, proteins were extracted using RIPA buffer (Sigma), and the amount of LC3-II was measured by Western blotting. In Western blotting, the membrane after protein transfer were blocked with 5% skim milk/TBST, and thereafter anti-LC3 antibody (Sigma, L7543) was added at 1:1,000 dilution, followed by shaking at 4°C overnight. Subsequently, HRP labeled anti-rabbit or mouse IgG antibody (GE Healthcare) was added thereto as a secondary antibody at 1:5,000 dilution, followed by shaking at room temperature for one hour. Bound antibodies were visualized using ODYSSEY Fc Imaging System (LICOR) through chemiluminescence with ECL prime (GE Healthcare). As an internal standa *β* rd, anti-β-actin antibody (Sigma) was used.

As typical examples, Figure 10a shows the results of Western blotting for LC3-II in the skin at the upper outer arm and elbow site of three test subjects. It was shown that the amount of LC3-II detected at the elbow site was low as compared with that in the upper outer arm, and the autophagic activity was further reduced at the elbow site. Also for the other two, the same results were obtained. Figure 10b shows quantitative values of autophagic activity for a total of the five test subjects. In the skin at the elbow site of AA, the autophagic activity was statistically significantly reduced to 30% or less as compared with that at the upper outer arm (paired t-test, p < 0.01).

Subsequently, the relationship of the quantified autophagic activity with the skin property values was analyzed. Statistically significant correlations of the autophagy with the skin saturation (C*), the stratum corneum water content, and the trans-epidermal water loss (TEWL) were observed (Figure 10c, Regression analysis, p < 0.05), and this fact suggested a possibility that the autophagic activity contributed to both the skin tone and the stratum corneum functions.

The causes for the reduction of the autophagic activity in the skin at the elbow site of AA were investigated. Since the autophagic activity is known to increase when mTORC1 signal is suppressed, mTORC1 activity in the skin collected above was evaluated. p70S6 kinase (p70S6K), which is phosphorylated by mTORC1, is used as one of indicators of mTORC1 activity. Therefore, the phosphorylated state of p70S6K in the skin was detected by Western blotting. In Western blotting, anti-phosphorylated p70S6K antibody (Cell Signaling Technology, #9234 or #9205) or anti-p70S6K antibody (Cell Signaling Technology, #2708) was used as a primary antibody. Other detection conditions were according to the aforementioned method. A signal specific to the phosphorylated p70S6K detected by Western blotting was quantified by image analyses and were evaluated. As a result, the phosphorylation of p70S6K was increased in the skin at the elbow site of AA (Figure 11a), and the increase was statistically significant as compared with that at the upper outer arm (Figure 11b, paired t-test, p < 0.05). Accordingly, it was suggested that autophagy was inhibited by mTORC1 activation in the skin at the elbow site of AA.

### [Example 4]

Example 3 suggested autophagy inhibition in the skin at the elbow site of AA via mTORC1 activation. Therefore, in this example, an ameliorating effect of a pharmaceutical agent for activating autophagy through mTORC1 inhibitory action, on uneven skin tone was investigated.

From skin at the elbow site of four AA women, skin tissues were collected through punch biopsy by a dermatologist. The collected tissues were halved with a scalpel, and the tissues were cultured for 5 days in the presence or absence of mTORC1 inhibitor, Torin 1 (Cayman Chemical, #10997) to a final concentration of 1 *µ*M. In the tissue culture, a culture medium obtained by adding 10v/v% Fetal Bovine Serum (FBS) and antibiotics (Antibiotic-Antimycotic) to Advanced-DMEM was used (both were from Life Technologies). After the completion of culture, frozen samples were rapidly produced, and immunohistochemical analyses were performed by the same procedure as in Example 1 (2) using their sections. Refer to Table 3 for the type of primary antibody, the tissue fixation conditions, and the concentration used. Further, melanin was visualized by Fontana-Masson staining, and the epidermal stratum corneum thickness and the melanin deposition area were calculated using an image analysis software (Image J).

**[Table 3]**

| Primary antibody used for immunohistochemical analyses | | | |
|---|---|---|---|
| Antibody | Vendor, Catalog No. | Fixation solution | Dilution |
| mTOR | Cell signaling, 2983S | Cold-Acetone | 1:50 |
| p-mTOR (Ser2448) | Cell signaling, 2976S | Cold-Acetone | 1:100 |
| LC3 | Cell signaling, 2775S | 4%PFA/PBS | 1: 100 |
| ATG5-ATG12 | Sigma, A0856 | 4%PFA/PBS | 1: 400 |
| ATG16L1 | LifeSpan, LS-B8297 | 4%PFA/PBS | 1: 300 |
| Filaggrin | Abcam, ab3137 | Cold-Acetone | 1: 500 |
| Loricrin | BioLegend, 905101 | Cold-Acetone | 1: 1,000 |
| Transglutaminase-1 (TGM1) | Novus, NB100-1844 | Cold-Acetone | 1: 400 |
| p62/SQSTM1 (p62) | MBL, M162-3 | 4%PFA/PBS | 1: 40 |
| Ki67 | DAKO, M7240 | 4%PFA/PBS | 1: 100 |

As a result, both the epidermal stratum corneum thickness and the amount of melanin statistically significantly decreased by the addition of Torin 1 (Figure 12, and Figure 13, paired t-test, p < 0.05). In the Torin 1-treated skin, inhibition of phosphorylation of mTOR was observed (Figure 12), and the inhibition was statistically significant (Figure 14 a, paired t-test, p < 0.05). Further, LC3 protein increased, and p62 protein aggregation decreased (Figure 12, and Figure 14b to c, paired t-test, p < 0.05). In the Torin 1-treated skin, localization of ATG5-ATG12 and ATG16L1, which are other autophagy-related proteins, from the basal layer to the spinous layer of the epidermis similar to that in the skin at the upper arm was observed, indicating that the localization was normalized (Figure 12). Further, in the Torin 1-treated skin as compared with untreated skin, distribution of keratinization marker proteins, Filaggrin (FLG), Loricrin (LOR), and Transglutaminase 1 (TGM1) in the epidermis was restored to be localized by the epidermal granular layer (Figure 12 and Figure 14d), and a decrease in proliferating cells was confirmed from the number of Ki67 positive cells (Figure 12).

### [Example 5]

Torin 1 was added to cultured keratinocytes derived from human neonatal foreskin, and the mixture was cultured for 24 hours in an environment of 37°C and 5% CO₂. For the cell culture, a culture medium obtained by adding a predetermined growth additive (Humedia KG2, manufactured by KURABO INDUSTRIES LTD.) to Epilife culture medium (Life Technologies) was used. RNA was extracted from the keratinocytes, and the gene expression was analyzed by quantitative PCR using a Taqman probe (Life Technologies). As a result, Torin 1 statistically significantly increased the gene expression of keratinization-related proteins, TGM1 and Involucrin (IVL), in the cells, depending on the dose thereof, to activate the differentiation of keratinocytes, while statistically significantly reducing the gene expression of SKP2, which is known to inhibit autophagy (Figure 15, ANOVA, Dunnett test, p < 0.05).

From these findings, a possibility that restoration of autophagic activity by the inhibition of mTORC1 not only can promote melanin metabolism in the epidermis but also can effectively ameliorate the uneven skin tone at joint sites which is specific to AA by normalizing the epidermal differentiation and ameliorating the stratum corneum functions.

### [Example 6]

The autophagy-activating actions of senrenshi extract and toosendanin which is the main component of senrenshi extract were investigated. The senrenshi extract was prepared by extraction from 10 kg of senrenshi (crude drug, manufactured by Tochimoto Tenkaido Co., Ltd.) with 100 L of a 70% ethanol aqueous solution, followed by treatment with activated carbon (CARBORAFFIN, manufactured by Japan EnviroChemicals, Limited) and subsequent solvent transfer into 80v/v% 1,3-butylene glycol. The prepared senrenshi extract had a solid content concentration of 0.5w/v%, and the toosendanin concentration in the extract was about 20 *µ*M.

To a keratinocyte culture medium prepared using neonatal foreskin purchased from an American tissue bank, National Disease Research Interchange (NDRI) (in accordance with the culture medium of Example 5), was added the senrenshi extract at a predetermined concentration or toosendanin (purchased from Ava Chem Scientific). At this time, the senrenshi extract-supplemented medium and the toosendanin-supplemented medium were adjusted to have the same toosendanin concentration in the culture medium. Only a solvent (ethanol) was added to the culture medium to serve as the control. The culture medium was cultured in an environment of 37°C and 5%CO₂ for 48 hours, and thereafter proteins were extracted from the cultured cells, to detect LC3-II protein in the cells by Western blotting (the detection procedure was in accordance with the method of Example 3). Figure 16 shows the results. The LC3-II protein-increasing actions of the senrenshi extract and toosendanin were confirmed. Further, since the LC3-II protein-increasing actions in these two substances were equivalent to each other, a possibility that the active component in the senrenshi extract was toosendanin was strongly suggested.

### [Example 7]

Melanosome degradation activity by the senrenshi extract was investigated. A melanosome fraction was prepared from melanocytes derived from human neonatal foreskin (manufactured by KURABO INDUSTRIES LTD.) according to a literature (Murase et al., J Invest Dermatol, 2013, 133: 2416-2424). To a culture medium of keratinocytes in which melanosome had been incorporated beforehand over three days (purchased and prepared in the same manner as in Example 5), was added the senrenshi extract, and the mixture was cultured for two days. After proteins were extracted from the cultured cells, Pmel17 protein serving as an indicator of melanosome was detected by Western blotting. In Western blotting, anti-Pmel17 antibody (DAKO, M0634) at 1:200 dilution was used as a primary antibody, and other conditions were in accordance with the method of Example 3. Figure 17 shows the results. The senrenshi extract promoted melanosome degradation depending on the dose.

### [Example 8]

The ameliorating action of the senrenshi extract on uneven skin tone was investigated. A lotion obtained by mixing 2.5%(v/v) of the senrenshi extract prepared in Example 6 with a base lotion containing 8.5%(v/v) glycerin was applied to either of the left and right of the arms and legs of twenty-one AA women, and a control lotion (placebo; obtained by mixing 80v/v% 1,3-butylene glycol, instead of the senrenshi extract, with the base lotion) was applied to the other thereof, twice a day for 8 weeks. Before the lotion application (Baseline) and after the application for 4 weeks and 8 weeks, the skin was photographed, and the skin tone was measured, by the same procedure as in Example 1 (1). Further, questionnaires were carried out after the application for 4 weeks and 8 weeks to check the awareness of amelioration in uneven skin tone by the test subjects.

Figure 18 shows the questionnaire results after the application for 4 weeks. A statistically significant difference was recognized in the awareness of amelioration in uneven skin tone by the test subjects to which the senrenshi extract mixed lotion was applied, as compared with that with the placebo (Wilcoxon signed rank test, p < 0.05).

The uneven tone levels of the test subjects were scored at five levels (1: I am not sure, 2: Very Even, 3: Somewhat Even, 4: Somewhat Uneven, 5: Very Uneven), and then five test subjects with the highest uneven tone level (Very Uneven) were selected. The effects of the senrenshi extract on the skin at the elbow site of these test subjects were investigated, based on the skin images and the measured skin tone values. As a result, the senrenshi extract statistically significantly ameliorated the saturation (C*) of the skin with uneven tone (Figure 19a, ANNOVA, Dunnett test, p < 0.05). Further, as a result of comparison of enlarged images of the skin surfaces of these test subjects between before and after the application, an ameliorating effect of the senrenshi extract on uneven skin tone, which was obvious also from the appearance, with amelioration in ashy skin and skin tone, was observed (Figure 19b).

From these results, it was suggested that a senrenshi extract which activates autophagy and promotes melanosome degradation, and toosendanin which is an active component of the senrenshi extract is effective for ameliorating uneven skin tone, such as ashy skin with pigmentation, which is often seen particularly in the skins over joint sites of African-descent.

## Claims

1. An in vitro method for evaluating or selecting an agent for preventing or ameliorating uneven skin tone, the method comprising:
administering test substances to cells;
measuring changes in autophagic activity caused by the test substances in the cells; and
selecting a test substance which has enhanced the autophagic activity.

2. A non-therapeutic method for preventing or ameliorating uneven skin tone, the method comprising: enhancing autophagic activity in keratinocytes in skin in need thereof, wherein the uneven skin tone is skin discoloration with skin dryness, thickening, and pigmentation, or ashy skin with pigmentation, wherein the uneven skin tone is uneven tone in skin over joints, and wherein the enhancement of the autophagic activity in keratinocytes in the skin comprises administering at least one selected from the group consisting of Melia toosendan, its extract, and toosendanin to the skin.

3. The non-therapeutic method according to claim 2, wherein the enhancement of the autophagic activity in keratinocytes in the skin comprises administering an autophagy inducer to the skin.

4. A non-therapeutic method for preventing or ameliorating uneven skin tone, the method comprising: administering at least one selected from the group consisting of Melia toosendan, its extract, and toosendanin to skin in need thereof, wherein the uneven skin tone is skin discoloration with skin dryness, thickening, and pigmentation, or ashy skin with pigmentation, wherein the uneven skin tone is uneven tone in skin over joints.

5. Non-therapeutic use of at least one selected from the group consisting of Melia toosendan, its extract, and toosendanin, for preventing or ameliorating uneven skin tone, wherein the uneven skin tone is skin discoloration with skin dryness, thickening, and pigmentation, or ashy skin with pigmentation, wherein the uneven skin tone is uneven tone in skin over joints.

## Patentansprüche

1. In-vitro-Verfahren zur Bewertung oder Auswahl eines Mittels zur Vorbeugung oder Verbesserung eines unebenmäßigen Hauttons, wobei das Verfahren umfasst:
das Verabreichen von Testsubstanzen an Zellen;
das Messen von Veränderungen der autophagischen Aktivität, die durch die Testsubstanzen in den Zellen verursacht werden; und
das Auswählen einer Testsubstanz, die die autophagische Aktivität gesteigert hat.

2. Nicht-therapeutisches Verfahren zur Vorbeugung oder Verbesserung eines unebenmäßigen Hauttons, wobei das Verfahren umfasst: das Steigern der autophagischen Aktivität in Keratinozyten in Haut, die dies benötigt, wobei der unebenmäßige Hautton eine Hautverfärbung mit Hauttrockenheit, Verdickung und Pigmentierung oder eine aschfahle Haut mit Pigmentierung ist, wobei der unebenmäßige Hautton ein unebenmäßiger Ton der Haut über Gelenken ist, und wobei das Steigerern der autophagischen Aktivität in Keratinozyten in der Haut das Verabreichen von mindestens einem, ausgewählt aus der Gruppe, bestehend aus Melia toosendan, dessen Extrakt und Toosendanin, an die Haut umfasst.

3. Nicht-therapeutisches Verfahren gemäß Anspruch 2, wobei das Steigern der autophagischen Aktivität in Keratinozyten in der Haut das Verabreichen eines Autophagie-Induktors an die Haut umfasst.

4. Nicht-therapeutisches Verfahren zur Vorbeugung oder Verbesserung eines unebenmäßigen Hauttons, wobei das Verfahren umfasst: das Verabreichen von mindestens einem, ausgewählt aus der Gruppe, bestehend aus Melia toosendan, dessen Extrakt und Toosendanin, an Haut, die dies benötigt, wobei der unebenmäßige Hautton eine Hautverfärbung mit Hauttrockenheit, Verdickung und Pigmentierung oder eine aschfahle Haut mit Pigmentierung ist, wobei der unebenmäßige Hautton ein unebenmäßiger Ton der Haut über Gelenken ist.

5. Nicht-therapeutische Verwendung von mindestens einem, ausgewählt aus der Gruppe, bestehend aus Melia toosendan, dessen Extrakt und Toosendanin, zur Vorbeugung oder Verbesserung eines unebenmäßigen Hauttons, wobei der unebenmäßige Hautton eine Hautverfärbung mit Hauttrockenheit, Verdickung und Pigmentierung oder eine aschfahle Haut mit Pigmentierung ist, wobei der unebenmäßige Hautton ein unebenmäßiger Ton der Haut über Gelenken ist.

## Revendications

1. Méthode in vitro pour évaluer ou sélectionner un agent destiné à prévenir ou à améliorer le teint irrégulier, la méthode comprenant :
l'administration de substances testées à des cellules ;
la mesure des changements dans l'activité autophagique causés par les substances testées dans les cellules ; et
la sélection d'une substance testée qui a amélioré l'activité autophagique.

2. La méthode non thérapeutique pour prévenir ou améliorer le teint irrégulier, la méthode comprenant :
l'amélioration de l'activité autophagique dans les kératinocytes de la peau qui en a besoin, dans laquelle le teint irrégulier est une décoloration de la peau accompagnée d'une sécheresse, d'un épaississement et
d'une pigmentation de la peau, ou une peau cendrée avec pigmentation, dans laquelle le teint irrégulier est un teint irrégulier au niveau des articulations, et dans lequel l'amélioration de l'activité autophagique dans les kératinocytes de la peau comprend l'administration à la peau d'au moins un élément choisi parmi le groupe comprenant Melia toosendan, son extrait et la toosendanine.

3. La méthode non thérapeutique selon la revendication 2, dans laquelle l'amélioration de l'activité autophagique dans les kératinocytes de la peau comprend l'administration d'un inducteur d'autophagie à la peau.

4. La méthode non thérapeutique pour prévenir ou améliorer le teint irrégulier, la méthode comprenant :
l'administration d'au moins un élément choisi parmi le groupe constitué de Melia toosendan, son extrait et la toosendanine à la peau qui en a besoin, dans laquelle le teint irrégulier est une décoloration de la peau accompagnée d'une sécheresse, d'un épaississement et
d'une pigmentation de la peau, ou une peau cendrée avec pigmentation, dans laquelle le teint irrégulier est un teint irrégulier au niveau des articulations.

5. Utilisation non thérapeutique d'au moins un élément choisi parmi le groupe comprenant Melia toosendan, son extrait et la toosendanine, en tant que composition cosmétique pour prévenir ou améliorer le teint irrégulier, dans laquelle le teint irrégulier est une décoloration de la peau accompagnée d'une sécheresse, d'un épaississement et d'une pigmentation de la peau, ou une peau cendrée avec pigmentation, dans laquelle le teint irrégulier est un teint irrégulier au niveau des articulations.
